# EUROPEAN PATENT APPLICATION

(11) **EP 1 178 304 A2**
(43) Date of publication of application: **06.02.2002**
(21) Application number: 01118620.2
(22) Date of filing: 02.08.2001
(51) Int. Cl.: G01N 27/419, G01N 27/407

(54) **Trace oxygen measuring apparatus and measuring method**

(30) Priority: 04.08.2000 JP 2000236844; 04.08.2000 JP 2000236964
(71) Applicant: NGK INSULATORS, LTD., Nagoya-City, Aichi Prefecture 467-8530 (JP)
(72) Inventor: Muroguchi, Akihiro, c/o NGK Insulators, Ltd., Nagoya-city, Aichi-prefecture, 467-8530 (JP); Mizutani, Yoshihiko, c/o NGK Insulators, Ltd., Nagoya-city, Aichi-prefecture, 467-8530 (JP)
(74) Representative: Leson, Thomas Johannes Alois, Dipl.-Ing.

(57) **Abstract**

The application discloses a trace oxygen measuring apparatus (40) capable of measuring accurately a concentration of a trace oxygen contained in various gases by using a limiting current type oxygen sensor having an improved output linearity within a region of the trace amount of oxygen, and taking the difference in pump current between the measurement gas from which oxygen has been removed and the measurement gas itself, and a measuring method using the same.
Furthermore, the present application relates to a device for generating oxygen in a trace amount (42); said device being usable for testing of the effect of trace oxygen, particularly to a device for generating oxygen in a trace amount usable as a device for calibration in a trace oxygen measuring apparatus usable for control of a manufacturing process of various industrial gases containing a trace combustible gas and trace oxygen therein, and a trace oxygen generating apparatus using said device for generating oxygen in a trace amount and a trace oxygen generating method using the same.

## Description

### BACKGROUND OF THE INVENTION

### 1. Field of the Invention

The present invention relates to a trace oxygen measuring apparatus usable for control of the manufacturing process of various industrial gases containing a trace combustible and trace oxygen therein and for quality control of various final products, and a measuring method thereof. More particularly, the present invention relates to a trace oxygen measuring apparatus capable of measuring accurately a concentration of a trace oxygen contained in various gases by using a limiting current type oxygen sensor having an improved output linearity within a region of the trace amount of oxygen, and taking the difference in pump current between the measurement gas from which oxygen has been removed and the measurement gas itself, and a measuring method using the same.

### 2. Description of the Related Art

Furthermore, the present invention relates to a device for generating oxygen in a trace amount; said device being usable for testing of the effect of trace oxygen, particularly to a device for generating oxygen in a trace amount usable as a device for calibration in a trace oxygen measuring apparatus usable for control of a manufacturing process of various industrial gases containing a trace combustible gas and trace oxygen therein, and a trace oxygen generating apparatus using said device for generating oxygen in a trace amount and a trace oxygen generating method using the same.

### STATE OF THE ART

High-purity gases are used in various fields including not only the gas refining industry, but also the semiconductor manufacturing process, heat treatment of steel and other metals (oxidation-free furnace), welding of special metals, and food packaging. Among these high-purity gases, in control of the manufacturing process of high-purity gases such as argon (Ar) gas, nitrogen (N₂) gas, or helium (He) gas, or quality control of the various final products, there is a demand for accurate measurement of oxygen concentration in a ppb order.

In general, theses high-purity gases including trace oxygen contain, in addition to this trace oxygen, a slight amount of combustible gases as impurities. For example, high-purity Ar gas is known to contain such combustible gases as CO of 0.1 ppm or less, CH₄ of 0.1 ppm or less, and H₂ of 0.2 ppm or less. Mixture of these impurities including trace oxygen is almost unavoidable in gases refined in a chilled air separator used for the manufacture of many industrial high-purity gases. The amount of mixed impurities has already reached the lowest level capable of being industrially removed.

The yellow phosphorus luminescent type and the special galvanic type oxygen sensors have been used for determining trace oxygen quantitatively in such high-purity gases. For a gas containing a combustible gas, magnetic type sensors have been used. These oxygen sensors have however various problems such as a high price, and in contrast, a short service life, and in addition, the necessity to make scrupulous maintenance and control. There is therefore a demand for emergence of an oxygen sensor being compact in size, easy in operation and maintenance, low in cost and long in service life.

A possible candidate for oxygen sensor capable of coping with such a demand is an oxygen sensor using zirconia (ZrO₂) exhibiting a satisfactory oxygen ion conductivity at high temperatures. In the ZrO₂ oxygen sensor, a ZrO₂ ceramic is held between metal electrodes and the measurement gas comes into direct contact with the metal electrodes. Therefore, if oxygen and a combustible gas coexist in the measurement gas, combustion of the combustible gas takes place on the metal electrodes, and this causes a problem of a decrease in oxygen concentration. That is, this results in detection of a concentration lower than the actual concentration of oxygen contained in the measurement gas.

It has been therefore the conventional practice to measure the residual oxygen concentration after consumption of oxygen on the metal electrodes, then, and making a correction by calculating subsequently an oxygen quantity necessary for combustion of the combustible gas on the basis of the chemical analysis concentration separately analyzed, thereby determining the initial concentration of oxygen present in the measurement gas. However, rapid measurement is impossible in this method, and because of combination of measured results derived from different measuring means, a decrease in measuring accuracy cannot be denied.

The present inventors have therefore proposed, in JP-A-2001-108651, a trace oxygen measuring apparatus capable of coping with a combustible gas using oxygen sensors having oxygen ion conductive solid electrolyte and metal electrodes separately as a bias sensor and a measure sensor, wherein, in the bias sensor, the quantity of combustible gas contained in the measurement gas is measured by burning the combustible gas contained in the deoxidized measurement gas obtained by passing the measurement gas through a deoxidizing column, by means of oxygen fed from the oxygen pump cell. In the measure sensor, the combustible gas contained in the measure gas is burned by sucking oxygen in a quantity corresponding to that of the combustible gas as measured by the bias sensor into the measurement gas by means of the oxygen pump cell, thereby measuring the concentration of oxygen contained in the measurement gas from the initial stage. Although it is possible to measure concentration up to units of ppb, there is not always available a high-linearity relationship between the output value of the measuring sensor and the oxygen concentration.

A commercially available standard gas for analysis, which is usually nitrogen gas contains, on the other hand, oxygen in a quantity of up to 0.5 ppm. This causes inconveniences when preparing a calibration curve for measurement in units of ppb. Therefore, in order to more accurately measure the oxygen concentration in unit of ppb, this is also a problem to be solved.

Among the other high-purity gases, as described above, there is tendency toward demanding a more accurate measurement of oxygen concentration in units of ppb in the control of manufacturing process of high-purity gases requiring control of oxygen concentration such as argon (Ar) gas, nitrogen (N₂) gas and helium(He) gas, and in quality control of the final products.
For this purpose, availability of reference oxygen gas having a ppb-level concentration is essential. However, on the present level of art, there are commercially available oxygen-mixed standard gases containing oxygen in an amount of at least 1 ppm. When preparing a standard oxygen gas having a ppb-level concentration under current circumstances, therefore, there are available only a method of mixing nitrogen gas substantially containing no oxygen with nitrogen gas containing oxygen in a known quantity of about 1 ppm by use of a mass flow controller, and another method of passing nitrogen substantially containing no oxygen in a prescribed quantity through a tube made of an oxygen-transmissive material such as a plastic tube or a rubber tube, causing oxygen in the open air to pass through, and obtaining a standard gas containing oxygen having a desired concentration by changing the tube length. In addition, there is available a method of diluting the oxygen concentration to a desired value by use of a gas separator.

On the other hand, there is partially used in the plant sites a method preparing a reference gas containing a predetermined amount of trace oxygen by adding a so-called standard gas containing 1 ppm oxygen to a zero gas such as nitrogen gas prepared by removing oxygen by means of a deoxidizer. The oxygen concentration in this case is calculated from the difference in the flow rate between the zero gas serving as a carrier gas and the standard gas containing 1 ppm oxygen.

The methods using a mass flow controller or oxygen-transmissive materials have problems in impossibility to perform accurate control of concentration of generated oxygen, and accuracy itself for a complicated operation.

On the other hand, in the method of adding oxygen in a prescribed quantity by adding a standard gas to zero gas prepared by removing oxygen by means of a deoxidizer, the ration of consumption differs between zero gas prepared by removing oxygen and the standard gas. The concentrations of combustible gas and other impurities in gas containing oxygen in a prescribed quantity may therefore vary, this forming a problem as a standard gas.

The commercially available standard gas having an oxygen concentration of 1 ppm has an effective number of digits of two. It is therefore problematic to use it for performing a precision measurement on the ppb level. In order to prepare a calibration gas having a wide oxygen concentration range of from 1 ppb to 1 ppm, it is necessary to scrupulously adjust the flow rate of standard gas containing 1 ppm oxygen, requiring four mass flow controllers including three controllers having different flow rate ranges for 1 ppm standard gas are one controller for zero gas not substantially containing oxygen. This inevitably leads to a larger-scale apparatus and a longer period of time for obtaining a calibration gas at a stable concentration.

### SUMMARY OF THE INVENTION

The present invention has been developed in view of the above-mentioned problems in the conventional art. It is an object of a first aspect of the present invention to provide a trace oxygen measuring apparatus and a measuring method capable of measuring rapidly and accurately a concentration of a trace oxygen by achieving a high linearity between output value of the measuring sensor and the oxygen concentration, with exclusion of the influence of an interference gas such as combustion of a coexisting combustible gas on the measured oxygen concentration, and making it possible to prepare a calibration curve for oxygen concentration measurement in units of ppb.

Oxygen concentration control in a space containing a concentration detecting electrode becomes more accurate by using a zirconia sensor as a measuring sensor, ascertaining that the electromotive force-oxygen concentration characteristic of a zirconia cell certainly follows up Nernst's formula within a range of at least 2 ppm, and setting a concentration detecting cell electromotive force which is feedback threshold value of a limiting current type oxygen sensor of up to 240 mV corresponding to oxygen concentration of at least 2 ppm. Along with this, oxygen in a quantity proportional to the difference between the measurement gas oxygen concentration and the set oxygen concentration is fed and discharged. In the trace oxygen concentration region, in which the relationship [measurement gas oxygen concentration] ≤ [set oxygen concentration] is valid, the oxygen is fed. A high correlation between the pump current and the fed/discharged oxygen quantity is kept by maintaining a high ion exchange ration by providing the oxygen feed electrode in the air duct in which the oxygen concentration is high. It was found possible to achieve the aforementioned object by providing a concentration detecting cell electrode and an oxygen pump electrode independently of each other so as to avoid the effect of voltage drop caused by pump current, providing an oxygen remover 41 to exclude the effect of interference gases such as coexisting combustible gases, calculating the concentration of oxygen contained in the measurement gas from the difference in measured value between one having passed through the oxygen remover 41 and one not having passed through the same, and incorporating a mechanism permitting preparation of a calibration curve for measuring the oxygen concentration in units of ppb. As a result of these findings, the first aspect of the present invention was developed.

More specifically, according to the first aspect of the invention, there is provided a trace oxygen measuring apparatus having at least one limiting current type oxygen sensor 10 having an oxygen pump cell 14 comprising an oxygen ion conductive solid electrolyte and metal electrode and a concentration detecting cell 13, which is applicable as a blank sensor or a measure sensor; wherein, when said limiting current type oxygen sensor 10 serves as a blank sensor, the oxygen concentration of deoxidizing measuring gas obtained by feeding a measurement gas through an oxygen remover 41 is measured by means of a limiting current type sensor; when said limiting current type oxygen sensor serves as a measure sensor, the oxygen concentration of the measurement gas is measured by means of pump current of the limiting current type sensor; and the apparatus has a mechanism 80 which calculates a difference in pump current between the measure sensor and the blank sensor as an oxygen concentration contained in the measurement gas.

There is provided also a trace oxygen measuring apparatus, having a branching mechanism which branches the measurement gas; wherein a measurement gas passes through the oxygen remover 41 by the action of the branching mechanism and is then fed to the blank sensor 52; and the other measurement gas is directly fed to the measure sensor 51 by the action of the branching mechanism. In this case, a switching mechanism may be provided, wherein action of the branching mechanism is switched over at a prescribed point in time; one of times is for feeding the deoxidized measurement gas after passing through the oxygen remover 41; and, during the other time, the measurement gas is fed to a particular oxygen sensor among at least one oxygen sensors, and there is provided a switching mechanism for measuring values of pump current as a blank sensor and a measure sensor.

According to the present invention, there is provided also a trance oxygen measuring apparatus having two limiting current type oxygen sensors each having an oxygen pump cell 14 comprising the oxygen ion conductive solid electrolyte and a metal electrode, and a concentration detecting cell 13, wherein one of the limiting current type oxygen sensor is used as a blank sensor 52, and the other limiting current type oxygen sensor is used as a measure sensor 51.

In any one of the above-mentioned trance oxygen measuring apparatuses, oxygen is fed or discharged by current energizing the oxygen pump of the limiting current type oxygen sensor, and there should preferably be provided a feedback controller 71 which controls the electromotive force of the concentration detecting cell 13 into a prescribed set voltage. The trace oxygen measuring apparatus may have a mechanism, in which the set voltage of the electromotive force of the concentration detecting cell 13 in the feedback controller 71 is controlled to a voltage of up to 240 mV which corresponds to an oxygen concentration range of at least 2 ppm ensuring followup of the electromotive force-oxygen concentration characteristics of the concentration detecting cell to Nernst's formula. It is also preferable that the trace oxygen measuring apparatus further comprises a special air duct communication with the open air as an oxygen source to maintain a high ion exchange rate an improve further the measuring accuracy.

According to the first aspect of the invention, furthermore, there is provided a trace oxygen measuring apparatus, wherein the oxygen sensor is formed with a plurality of solid electrolyte layers; an a first air duct 12A, a second air duct 12B, and a measuring duct 19 defined by the plurality of solid electrolyte layers; the measuring duct 19 has an oxygen discharge electrode 16 and a concentration detecting electrode 17; an oxygen pump cell formed of an oxygen feed electrode formed in the first air duct 12A, and an oxygen discharge electrode formed in the measuring duct 19 via the solid electrode layers formed between the first air duct 12A and the measuring duct 19; and a concentration detecting cell having an air reference electrode 18 formed in the second air duct 12B and a concentration detecting electrode 17 formed in the measuring duct, via the solid electrolyte layers formed between the second air duct 12B and the measuring duct 19; and a mechanism for measuring the oxygen concentration in the measurement gas by measuring the oxygen pump current during feedback control through operation of the oxygen pump so that the electromotive force of the concentration detecting cell becomes a prescribed set voltage.

In this trace oxygen measuring apparatus, since the oxygen pump cell 14 and the concentration detecting cell 13 have the same configuration, it is possible to replace these cells with each other, the electrode presenting in the first air duct in the oxygen sensor being used as a reference electrode, the opposite electrode being used as a detecting electrode, the electrode present in the second air duct being used as an oxygen feed electrode 15, and the opposite electrode thereto being used as an oxygen discharge electrode 16.

Furthermore, there is provided a method of measuring the trace oxygen concentration in a measurement gas containing trace oxygen by means of an oxygen sensor, comprising the steps of using the oxygen sensor, wherein the oxygen sensor is formed with a plurality of solid electrolyte layer; and a first air duct 12A, a second air duct 12B and a measuring duct 19 defined by the plurality of solid electrolyte layers; the measuring duct 19 has an oxygen discharge electrode 16 and a concentration detecting electrode 17; and oxygen pump cell 14 formed of an oxygen feed electrode 15 formed in the first air duct 12A, and an oxygen discharge electrode 16 formed in the measuring duct 19 via the solid electrolyte layers formed between the first air duct 12A and the measuring duct 19; a concentration detecting cell 13 having an air reference electrode 18 formed in the second air duct 12B and a concentration detecting electrode 17 formed in the measuring duct 19, via the solid electrode layers formed between the second air duct 12B and the measuring duct 19. The accuracy in the detection of the oxygen concentration is improved and output linearity in the trace oxygen region is obtained, thereby permitting very accurate measurement by using an oxygen sensor having a configuration of feedback-controlling the electromotive force of the concentration detecting cell 13 to a prescribed set voltage by means of a function of pumping an oxygen quantity corresponding to a pump current value of the oxygen pump cell 14 from the first air duct 12A into the measuring duct 19, using the oxygen sensor which measures the oxygen concentration in the measurement gas from the pump current of the pump cell, and controlling the feedback control set voltage of the oxygen sensor to a voltage of up to 240 mV which corresponds to an oxygen concentration range of at least 2 ppm ensuring followup of Nernst's formula. In this measuring method, the relationship [measurement gas oxygen concentration] ≤ [set oxygen concentration] is valid within the trace oxygen range, and the pump current is directed in the oxygen pumping direction into the measuring duct. Because the oxygen flow from the measuring duct 19 or the second air duct 12B leads to a decrease in ion exchange rate caused by the reduction of oxygen concentration, the first air duct 12A is provided as an oxygen feed air duct communicating with the specific open air. Oxygen feed therefrom gives a satisfactory linearity of the pump current output/oxygen concentration characteristics, and permit accurate measurement of the oxygen concentration on a ppb-level.

A second aspect of the invention has been developed in view of the problems in the conventional art regarding the above-mentioned trace oxygen generating apparatus and the generating method, and has an object to provide a device for generating oxygen in a trace amount as a compact device capable of feeding accurately a calibrating gas containing oxygen on ppb-level, a trace oxygen generating apparatus using said device for generating a trace oxygen, and a method for generating a trace oxygen in such a manner as mentioned above.

It has been found that the above-mentioned object may be achieved by providing a variable constant current controller which operates the oxygen pump so as to permit generation of oxygen in an arbitrary quantity within a range of from 1 ppb to 2 ppm in the oxygen pump comprising a zirconia solid electrolyte cell, and an oxygen feed electrode and an oxygen discharge electrode formed on the cell, and the present invention was thus developed.

More specifically, according to the second aspect of the invention, there is provided a device for generating oxygen in a trace amount comprising a plurality of solid electrolyte layers, and further comprising:
an oxygen feed duct comprising a first duct, which is a vacancy defined by the solid electrolyte layers forming three continuous layers a least at an end thereof and an oxygen feed electrode formed in the first air duct; and an oxygen pump cell comprising an oxygen discharge electrode provide on the surface of an uppermost layer of the solid electrolyte layers forming the aforementioned three layers and an oxygen feed electrode formed in the first air duct; and a constant current source/controller is arranged between the discharge electrode and the oxygen feed electrode so that prescribed current flows therethrough.

There is also provided a device for generating oxygen in a trace amount having, in addition to the first air duct, a second air duct and an air reference electrode provided in the second air duct; wherein the air reference electrode forms a detecting cell which monitors a decrease in oxygen concentration caused by oxygen feed in the oxygen feed duct through a charge in electromotive force by measuring electromotive force between the air reference electrode and the oxygen feed electrode in the oxygen feed duct.

A trace oxygen generating apparatus using any one of the aforementioned devices for generating a trace oxygen generating is also provided.

There is provided a method of generating trace oxygen, comprising the steps of feeding constant current to the oxygen pump cell comprising an oxygen feed electrode and an oxygen discharge electrode by activating a constant current source/controller so as to generate prescribed oxygen and operating an oxygen pump; sending a gas from a carrier gas source to the oxygen pump; receiving oxygen fed from the oxygen pump; and feeding a carrier gas added with the resultant trace oxygen in a prescribed amount to a necessary point.

There is furthermore provided a method of generating trace oxygen comprising the steps of feeding constant current to an oxygen pump cell comprising an oxygen feed electrode and an oxygen discharge electrode by operating a constant current source/controller so as to generate prescribed oxygen, and operating an oxygen pump; sending zero gas which is a carrier gas from a carrier gas source from which oxygen has been removed through an oxygen remove using a deoxidizer; receiving oxygen fed from the oxygen pump; and feeding a gas containing trace oxygen in a resultant prescribed amount to places requiring such gas.

There is also provided method of generating trace oxygen comprising the steps of operating a constant current source/controller, and operating an oxygen pump by feeding constant current to an oxygen pump cell comprising an oxygen feed electrode and an oxygen discharge electrode so as to generate prescribed oxygen; adding oxygen in a slight amount to a carrier gas from a carrier gas source, heating the resultant mixture to burn the combustible fraction, removing oxygen in an oxygen remover using a deoxidizer or the like, and sending a zero gas from which the combustible fraction and oxygen have been removed to the oxygen pump side; receiving oxygen fed from the oxygen pump; and feeding a gas containing trace oxygen in a resultant prescribed amount to places requiring such gas.

### BRIEF DESCRIPTION OF THE DRAWINGS

Fig. 1 is a sectional view illustrating an embodiment of a ZrO₂/oxygen sensor suitably used in the trace oxygen measuring apparatus of the present invention;
Fig. 2 is a descriptive view illustrating an embodiment of the configuration of the trace oxygen measuring apparatus of the invention;
Fig. 3 is a block diagram illustrating an embodiment of the configuration of the trace oxygen measuring apparatus of the invention shown in Fig. 2;
Fig. 4 is a description view illustrating a configuration of another embodiment of the trace oxygen measuring apparatus of the invention;
Fig. 5 is a block diagram illustrating the configuration of the trace oxygen measuring apparatus of the invention shown in Fig. 4;
Fig. 6 is a descriptive view illustrating an example of configuration of the device for generating oxygen in a trace amount used in the trace oxygen measuring apparatus of the invention;
Fig. 7(a) (a), (b), (c), (d), (e) and (f) show graphs illustrating the relationship of differences in pump current output and output current of blank sensor and measure sensor as measured by the oxygen measuring apparatus of the invention and the oxygen concentration;
Fig. 8 is a descriptive view illustrating the form of operating of the oxygen sensors shown in Fig. 1;
Fig. 9 is a schematic view illustrating the configuration of the device for generating a trace oxygen of the second aspect of the invention;
Fig. 10 is a schematic view illustrating the configuration of the device for generating a trace oxygen having an oxygen concentration reduction detecting function of the second aspect of the invention;
Fig. 11 is a descriptive view illustrating an embodiment of the configuration of the trace oxygen measuring apparatus of the first aspect of the invention using the device for generating a trace oxygen of the second aspect of the invention;
Fig. 12 is a descriptive view illustrating another embodiment of the trace oxygen measuring apparatus of the first aspect of the invention in which oxygen is added to the carrier gas using the device for generating a trace oxygen of the second aspect of the invention;
Fig.13 is a description view illustrating another embodiment of the configuration of the trace oxygen measuring apparatus of the first aspect of the invention in which combustible a gases contained in carrier gas have removed using the device for generating a trace oxygen of the second aspect of the invention;
Fig. 14 is a block diagram illustrating an embodiment of the configuration of the trace oxygen measuring apparatus of the first aspect of the invention using the device for generating a trace oxygen of the second aspect of the invention shown in Fig. 11;
Fig. 15 is a flowchart illustrating the operating procedure of trace oxygen generating by the device for generating a trace oxygen of the second aspect of the invention shown in Fig. 9;
Fig. 16 present views representing the relationship between constant current at a carrier gas flow rate of 0.74 ml/min and the generated oxygen concentration (ppb): 16(a) illustrates O₂ concentration-pumping current characteristics when generating oxygen in a concentration varying from 0 to 1,140 ppb; and 16(b) illustrates O₂ concentration-pumping current characteristics when generating oxygen at a concentration varying from 0 to 50 ppb from among those shown in 16(a).

### DESCRIPTION OF THE PREFERRED EMBODIMENTS

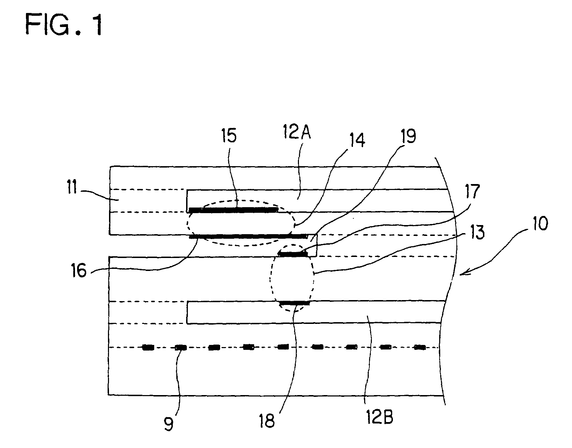

The trace oxygen measuring apparatus of the first aspect of the present invention (hereafter sometimes referred to as "measuring apparatus") is composed of a plurality of oxygen ion conductive solid electrolyte layers and uses an oxygen sensor comprising metal electrodes provide in a plurality of vacancies defined by the solid electrolyte layers. More specifically, the trace oxygen measuring apparatus comprises an oxygen ion conductive solid electrolyte and metal electrodes, and uses a limiting current type oxygen sensor which in an oxygen pump cell having a concentration detecting cell 13. Fig.1 is a sectional view illustrating the structure of the oxygen sensor 10 suitably used in the measuring apparatus of the present invention. There are formed on the oxygen sensor 10 a first air duct 12A defined by the plurality of solid electrolyte layers, a second air duct 12B and a measuring duct 19. Usually, the plurality of solid electrolyte layer forming the basic skeleton of a sensor an made, for example, from a zirconia (ZrO₂) ceramic 11 as shown in Fig. 1. A concentration detecting electrode 17 and an oxygen discharge electrode 16 are provided in the measuring duct 19. Air is fed to the first and second air ducts 12A and 12B, and a measurement gas is fed to the measuring duct 19.

The ZrO₂ porcelain 11 quite naturally serves as a solid electrolyte, and also plays the role of a partition of separating and regulating the first and second air duct 12A and 12B and the measuring duct 19. The solid electrolyte should preferably have a high oxygen ion conductivity. ZrO₂ allows changing the ion conductivity by means of the kind and quantity of a solute element. One having a composition suitable for purpose can therefore appropriately be used.

More specifically, as a ZrO₂ porcelain 11, a stabilized ZrO₂ or partially stabilized ZrO₂ made by solute processing various stabilizes such as yttria (Y₂O₃), magnesia (MgO), Calcia (CaO) and Ceria (CeO₂) is suitably applicable. Reduction of resistance between electrodes can be achieved by using a smaller thickness of the ZrO₂ porcelain between electrodes.

An oxygen feed electrode 15 formed on the first air duct 12A and an oxygen discharge electrode 16 formed on the measuring duct 19 as a pair form an oxygen pump cell 14. What is important here is that the configuration is such that a sufficient amount of oxygen is present in the first air duct 12A. When a electromotive force higher than a predetermined electromotive force in accordance with Nernst's formula is imparted to the oxygen concentration in the measurement gas, oxygen in fed to the measuring duct 19 by use of the above-mentioned oxygen pump cell 14 to replenish oxygen so that voltage detected at the concentration detecting electrode 17 and the air reference electrode 18 results in a prescribed electromotive force, and current flowing between the oxygen feed electrode 15 and the oxygen discharge electrode 16 formed on the measuring duct 19 becomes the pump current. A high linearity is thus available even in, for example, the region of under 2 ppm as described later. On the other hand, the pair of the concentration detecting electrode 17 formed in the measuring duct 19 and the air reference electrode 18 formed in the second air duct 12B forms a concentration detecting cell 13, and used for detecting the oxygen concentration in the measuring duct 19.

Then electrodes are required to have satisfactory electron conductivity, and a high oxygen ion catalytic property is also an important factor. For example, the oxygen ion catalytic property in the oxygen feed electrode 15 is defined as a property of ionizing oxygen molecules in the air and incorporating ions into a solid electrolyte. On the other hand, the oxygen ion catalytic property in the oxygen discharge electrode 16 means a property, in contrast, of taking up electrons from the oxygen ions moving in the solid electrolyte from the oxygen feed electrode 15 and releasing them as oxygen molecules to the measuring duct 19.

As an electrode material excellent in such a property, platinum (Pt) is suitably applicable. These electrodes should preferably be porous and have many triple points (three-phase interfaces) where the gas phase, the electrode and the solid electrolyte are in contact. Therefore, a thermet electrode comprising Pt and ZrO₂ is also suitably applicable.

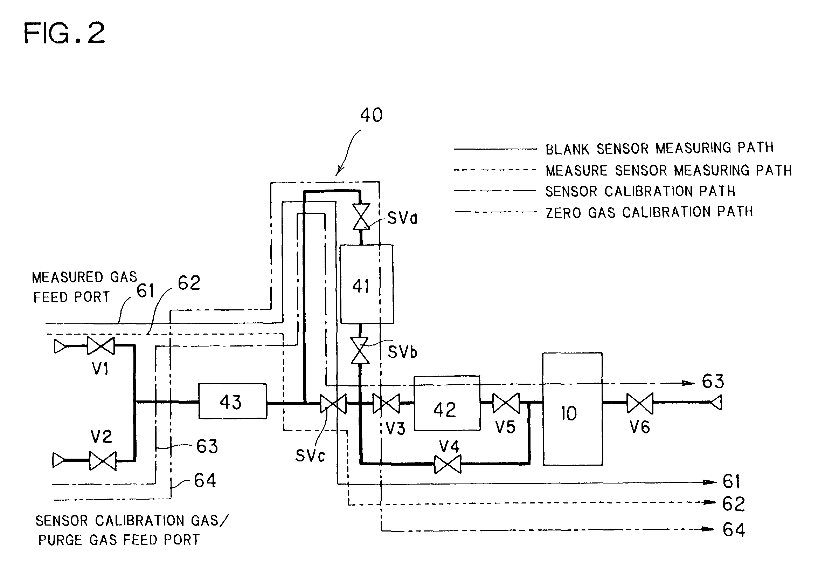

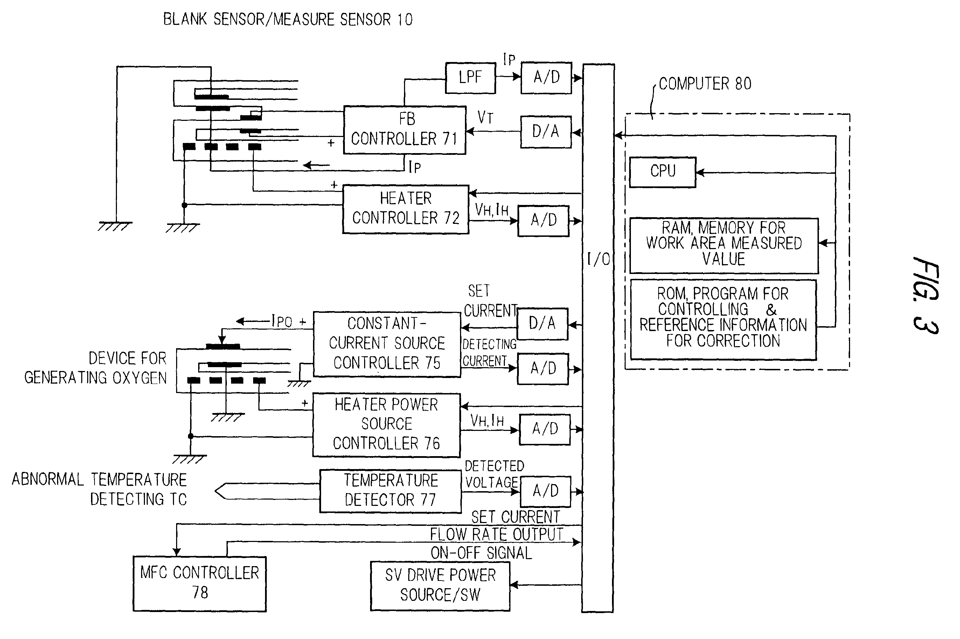

Although not shown in Fig. 2, but as shown in Fig. 3, a heater 9 heated by a heating unit configured so as to be capable of holding a prescribed temperature is arranged on the ZrO₂ porcelain 11. The ion conductivity of the ZrO₂ porcelain between electrodes is improved by holding a prescribed temperature through heating of the oxygen sensor 11 by the heater 9, this leading to a lower resistance between electrodes and hence improvement of the measuring accuracy.

Fig. 2 gives a schematic view for illustrating an outline of a configuration of the measuring apparatus 40 of the invention. In this embodiment, only one limiting current type oxygen sensor is provided, and this oxygen sensor 10 serves as both a blank sensor and a measure sensor. More specifically, when the limiting current type oxygen sensor serves as a blank sensor, the oxygen concentration of the deoxidation measuring gas obtained by passing the measurement gas through the oxygen remove 41 would be measured with pump current of the limiting current type sensor, and when the limiting current type oxygen sensor is used as a measure sensor, the oxygen concentration of the measurement gas would be measured with pump current of the limiting current type sensor. Calculation of the oxygen concentration is accomplished by using a mechanism which calculate the difference in pump current between the measure sensor and the blank sensor as the concentration of oxygen contained in the measurement gas, as described later. Fig. 3 is a block diagram of major components of the measuring unit 40 shown in Fig. 2.

In this embodiment, as shown in Fig. 2, in addition to the oxygen sensor 10 serving as both the blank sensor and the measure sensor, a measurement gas feed port playing the role of a branching mechanism branching the measurement gas, a mass flow controller 43, valves SVa, SVb, SVc and valves V1 to V6 are provided. The oxygen remover 41 is provided for feeding oxygen-removed measurement gas which the oxygen content in the measurement gas is brought substantially to zero on the oxygen sensor serving as the blank sensor. Furthermore, a sensor calibrating/purge gas feed port, and a device for generating oxygen 42 for adjusting the oxygen concentration in the calibrating gas to a prescribed value when feeding the sensor calibrating gas are provided. As shown in Fig. 3, an FB controller 71 in the oxygen sensor 10 so that the electromagnetic force of the concentration detecting cell 13 is always a prescribed voltage. For the purpose of excluding a measuring error caused by a change in temperature, a heater controller 72 for controlling the sensor to a prescribed temperature may be provided. Voltage corresponding to the pump current is output from the first and second FB controllers 71 and 71a so as to be able to detect pump current in a form in response to functioning as the measure sensor 51 and the blank sensor 52, respectively. The values of output voltage are converted into digital signals, sent to a computer 80, and computed so that the difference in pump current between the measure sensor 51 and the blank sensor 52 represents the concentration of oxygen contained in the measurement gas. That is, the FB controller 71 operates the oxygen pump and feedback-controls the electromotive force of the concentration detecting cell 13 so as to become a prescribed set voltage, converts the then oxygen pump current into a digital signal, sends the signal to the computer 80, and calculates the concentration of oxygen in the measurement gas on the basis of a prescribed program, thereby measuring the concentration of oxygen in the measurement gas.

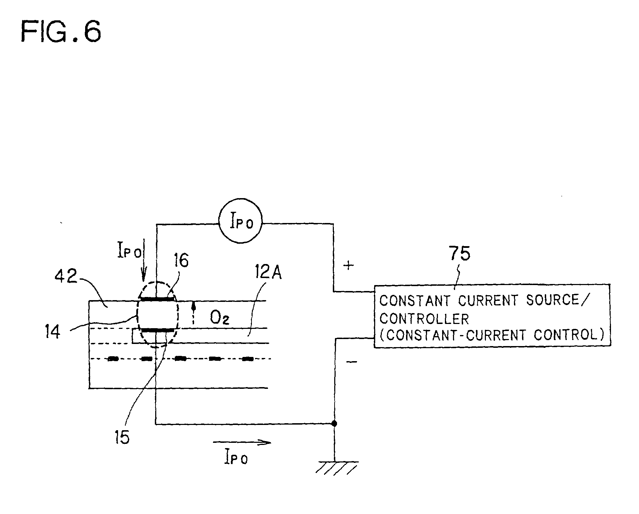

A sectional view of the basic structure of the device for generating oxygen 42 which is the apparatus for adding oxygen in a prescribed concentration to the standard gas used for calibration is illustrated in Fig. 6. As is clear from Fig. 6, the device for generating oxygen 42 comprises a solid electrolyte and a pair of electrodes and has an oxygen pump cell comprising an air duct which is a vacancy defined by the solid electrolyte, an oxygen feed electrode formed in the air duct, and an oxygen electrode provided on the surface of the solid electrolyte exposed to the measurement gas. A constant current source/controller is arranged between the oxygen discharge electrode and the oxygen feed electrode so as to feed desired constant current. As the device for generating oxygen 42 having such a configuration, the oxygen generating apparatus for generating oxygen of the second aspect of the invention described later is suitably applicable. A constant current source/controller 75 operable in response to an instruction from the computer 80 for controlling the quantity of generated oxygen, and a heater power source/controller 76 for controlling temperature of the device for generating oxygen to a prescribed temperature are provided on the device for generating oxygen 42.

For the purpose of keeping uniform measuring conditions, mass flow controllers 43 and 43a and an MFC controller 78 for controlling the flow rate of the measurement gas and the sensor calibrating/purge gas on a certain level are provided. As a safety unit for the apparatus, a temperature detector 77 which monitors presence or absence of occurrence of an abnormal temperature in the apparatus may be provided. The computer 80 comprises a CPU for performing computation for achieving command set, an RAM for work memory temporarily storing results of various measurements, and an ROM being installed with various programs controlling various parts and elements of the measuring unit in accordance with the functions thereof and storing reference information for correction.

In the measuring unit 40, a measurement gas feed port and a feed port of nitrogen gas which is a sensor calibrating/purge gas are provided at leading ends of a pipe having an end branching into two. These ports lead to a flow path via valves attached to the respective flow paths. In this configuration, the measurement gas is directly introduced into a flow path 62, under control of the MFC controller 78, via the mass flow controller 43 through prescribed switching of a series of valves V1 to V6, SVa, SVb and SVc, or directed through the oxygen remover 41 to the sensor, i.e., via the flow path 61 and via the oxygen remover, to reach the sensor 10. The standard gas used for sensor calibration, usually nitrogen gas also serving as a purge gas flows through the mass flow controller 43 so as to contain oxygen in a desired concentration, is directed to the oxygen remover 41 by switching of the valves SVa, SVb and SVc. In the oxygen remover 41, oxygen is removed to an oxygen concentration of under 1 ppb. Then, the gas is sent through a zero gas flow path 64 via the valve V4 to the oxygen sensor, or, by opening the two valves provided before and after the device for generating oxygen 42 and closing the bypass valve V4, passes through the device for generating oxygen 42 and adjusted to contain oxygen of a desired concentration of up to 2 ppm. The gas is finally sent to the oxygen sensor 10 via the flow path 63.

Commercially available nitrogen gas used as a sensor calibrating/purge gas usually contains up to 0.5 ppm oxygen. When an oxygen content of at least 2 ppm in the measurement gas is predicted, the valves SVa and SVb are closed to avoid premature wear of the oxygen remover 41. It suffices to configure the oxygen remover 41 with columns filled with a deoxidizer. An example of the deoxidizer is GASCLEAN (commercial product name).

Operation of the branching mechanism is switched over in terms of time. During a time, the deoxidized measurement gas having passed through the oxygen remover 41 is fed to the oxygen sensor. During the other time, the measurement gas is fed to the oxygen sensor. As a switching mechanism for measuring pump current between the blank sensor and the measure sensor by time, a switching valve operating in accordance with a program previously incorporated into the computer 80 so as to automatically perform switching of a series of valves shown in Fig. 2 every prescribed time, as shown in the block diagram given in Fig. 3 may be used. A timer mechanism in which the valves are automatically switched over at prescribed time intervals in response to the flow of gas may also be used. It is needless to mention that switching may be conducted in accordance with a factor other than time. In this case, for example, the valves may be opened or closed in response to the prescribed kind of the gas.

In order to more accurately measure the trace oxygen concentration, a feedback controller 71 based on current of the limiting current type oxygen pump cell should preferably be provided to bring the electromotive force of the concentration detecting cell 13 to a prescribed voltage. In this case, it is desirable to previously incorporate a necessary program into the computer 80 so as to permit setting and control of the set voltage of electromotive force of the concentration detecting cell 13 in the feedback controller 71 to a voltage of up to 240 mV which corresponds to an oxygen concentration range of at least 2 ppm ensuring followup of the electromotive force - oxygen concentration characteristics of the concentration detecting cell 13. A control mechanism should preferably be provided to inhibit influence of a large variation of the concentration in the measurement gas. As such a control mechanism, it is desirable to provide a special air duct 12A communicating with the open air and an oxygen pump cell 14 comprising an oxygen feed electrode 15 and an oxygen discharge electrode 16 having a function of pumping out oxygen from this duct preferably in the oxygen sensor 10, operate this oxygen pump and control the oxygen concentration within the measurement gas duct so as to satisfy the relationship [Measurement gas oxygen concentration] ≤ [Control set oxygen concentration].

When using a measuring apparatus in which an oxygen sensor 10 as shown in Fig. 2 serves simultaneously as a blank sensor and a measure sensor, control should be conducted by feeding the deoxidized measurement gas through the flow path 61 to the oxygen sensor 10, using the oxygen sensor 10 as a blank sensor, and performing control by feeding pump cell current I_{PB} so that the electromagnetic force of the concentration detecting cell 13 becomes a prescribed voltage of, for example, 210 mV on the basis of an instruction from the computer 80, by means of an FB controller 71 which is a mechanism which controls the electromotive force of the concentration detecting cell 13 to be the prescribed voltage. The prescribed voltage should preferably be 240 mV or under, which is the voltage corresponding to an oxygen concentration range of at least 2 ppm permitting maintenance of oxygen concentration in the measuring duct at a level higher than that of the measurement gas. The reason is that the oxygen partial pressure electromotive force of zirconia solid electrolyte cell usually causes an electromotive force satisfying Nernst's formula, and an oxygen concentration of 2 ppm becomes substantially the limit due to gas adsorption of the electrode and the like. The electromotive force of an oxygen concentration under 1 ppm is generally outside the acceptable range on the lower side. By limiting the set value of feedback control an electromotive force within a range ensuring followup of Nernst's formula, it is possible to accurately control the oxygen concentration within the measuring duct, thus permitting strict retention of a linear relationship between the oxygen concentration in the measurement gas and the pump current, and there is available a high output linearity of the trace oxygen concentration region in the limiting current type oxygen sensor.

Then, the flow path is switched over, and the measurement gas is fed to the oxygen sensor 10 through the flow path 62 leading directly to the oxygen sensor. Using the oxygen sensor 10 as the measure sensor, a pump current I_{PM} for achieving an electromotive force of the concentration detecting cell 13 of a prescribed voltage instructed from the computer 80 of, for example, 210 mV is determined by the FB controller 71. When calculating the oxygen concentration in the measurement gas, nitrogen gas which is the sensor calibrating/purge gas is fed into the flow path 63. The pump current at each of various oxygen concentrations in the deoxidized nitrogen gas is measured, and a calibration curve (relational formula) is prepared from the resultant values.

For the measured pump current upon measurement gas' having passed through the flow paths 61 and 62, and sensor calibrating/purge gas' having passed through the flow paths 63 and 64, a voltage signal corresponding to a pump current is issued from the FB controller 71 shown in Fig. 3. The issued voltage signal is converted into a digital signal, which is sent to the computer 80, and recorded, computing-processed and stored as blank sensor, measure sensor and sensor calibration values. The concentration of oxygen contained in the measurement gas is obtained as a difference between the blank sensor measured value.

In the measuring unit 40, therefore, use of a single oxygen sensor 10 as the measure sensor and the blank sensor eliminates dispersions caused by differences between the individual sensors, and leads to an advantage of a simple configuration. Pressure loss resulting from passage through the oxygen remover 41 is automatically adjusted by the mass flow controller 43.

The trace oxygen measuring apparatus shown in Fig. 2 comprises a measured gas feed port, a sensor calibrating gas/purge gas feed port, a mass flow controller, an oxygen remover 41, a device for generating oxygen 42, and an oxygen sensor 10, and is characterized in that it is composed of the first mass flow controller and a series of changeover valves so as to permit switching to and from the measured gas, the sensor calibrating gas and the blank gas.

Because the oxygen sensor serves also as a blank sensor and a measure sensor, the measured gas does not pass through the oxygen remover 41 or the device for generating a trace oxygen 42: the blank gas passes only through the oxygen remover 41, and the sensor calibrating gas passes through the oxygen remover 41 and the device for generating oxygen 42. The first mass flow controller 43 and the series of changeover valves are arranged so as to feed these gases into the first oxygen sensor 10. A feedback controller 71 should preferably be provided for controlling the electromotive force of the detecting cell provided on the oxygen sensor to a prescribed voltage.

In the trace oxygen measuring apparatus, the device for generating oxygen 42 may comprise a solid electrolyte and a pair of metal electrodes. It may have an oxygen pump cell 14 comprising an oxygen feed duct 12A comprising an air duct which is a vacancy defined by the solid electrolyte, and an oxygen feed electrode 15 formed on the surface of the solid electrolyte in the air duct, an oxygen discharge electrode 16 provided on the surface of the solid electrolyte exposed to the gas, and an oxygen feed electrode 15 formed in the air duct 12A. A constant current source/controller 71 may be arranged so that prescribed current flows between the oxygen discharge electrode 16 and the oxygen feed electrode 15.

In the trace oxygen measuring apparatus of the invention, the oxygen sensor may be formed with a plurality of solid electrolyte layers; and a first air duct 12A, a second air duct 12B and a measuring duct 19 defined by the plurality of solid electrolyte layers; the measuring duct may have an oxygen discharge electrode 16 and a concentration detecting electrode 17; an oxygen pump cell 14 formed of an oxygen feed electrode 15 formed in the first air duct 12A, and an oxygen discharge electrode 16 formed in the measuring duct 19 via the solid electrolyte layers formed between the first air duct 12A and the measuring duct 19; and a concentration detecting cell 17 having an air reference electrode 18 formed in the second air duct 12B and a concentration detecting electrode formed in the measuring duct 19, via the solid electrolyte layers formed between the second air duct 12B and the measuring duct 19; and a mechanism for measuring the oxygen concentration in the measurement gas by measuring the oxygen pump current during feedback control through operation of the oxygen pump so that the electromotive force of the concentration detecting cell 13 becomes a prescribed set voltage.

An example of measuring procedure in the measuring unit 40 shown in Fig. 2 will now be described. The measuring procedure comprises the steps of opening a purge gas feed port valve V2, starting the unit by turning on power, purging the flow paths with nitrogen gas, then, causing the oxygen generator 42 to heat-run, and at the same time, causing the oxygen sensor 10 and the drive FB controller 71 to heat-run. Subsequent steps comprise closing the valves V1, SVc and V4, opening the valves V2, SVa, SVb, V3, V5 and V6, and feeding deoxidized nitrogen gas via the flow path 63. When adding oxygen in a prescribed quantity to the deoxidized nitrogen gas, current corresponding to the desired quantity of oxygen is calculated by the constant current source/controller 75 and fed. Outflow of the standard gas containing prescribed oxygen into the flow path 63 is confirmed from, for example, the time lapse and the constant state of current flowing through the oxygen sensor 10, and the subsequent steps comprise recording the pump current which is an output signal of the oxygen sensor 10 in the computer 80, storing the same after computation, and calibrating the relationship between the oxygen concentration and the pump current of the oxygen sensor 10.

Then, supply of measurement gas is started by closing the valves V2, SVc, V3 and V5, opening the valves V1, SVa, SVb, V4 and V6, and causing the gas to pass through the flow path 61. After confirming that the gas flowing through the flow path 61 has been completely replaced by the deoxidized measurement gas, the oxygen concentration is detected by the oxygen sensor 10, and the result of detection is stored in the RAM as a blank sensor measured value. Then, the valve SVc is opened, and the valves SVa and SVb are closed. The measurement gas is fed through the flow path 62 into the oxygen sensor 10. After confirming that the gas flowing through the flow path 62 has been completely replaced by the measurement gas, the oxygen concentration is detected by the oxygen sensor 10, and the result of detection is stored in the RAM as a measure sensor measured value. The oxygen concentration of the measurement gas is determined by calculating the difference between the measure sensor measured value and the blank sensor measured value stored in the ROM by means of the computer 80. As required, it is also possible to calculate the combustible gas reducing concentration as converted into oxygen equivalent from the blank sensor measured value. Finally, the process is completed by turning off power.

When it is necessary to continuously measure oxygen in the measurement gas, the process may comprise repetition of only the steps from closing the valves V2, SVa, SVb, V3 and V5, and opening the valves V1, SVc, V4 and V6, up to the calculation in compliance with the instruction of the computer 80. When correction is necessary, it is accomplished by use of a correcting program of ROM.
Operation is usually performed by a program previously incorporated into the computer 80. The aforementioned operating procedure is only an example, and it is possible to appropriately modify or change the procedure in response to the kind of measurement gas, measuring environment and object of measurement. It is needless to mention that even such a change can be efficiently coped with by previously incorporating a prescribed program into the computer. When it is clear that there is no change in the measuring conditions, it is of course possible to previously incorporate a prescribed program so as to omit sensor calibrating operation using nitrogen gas.

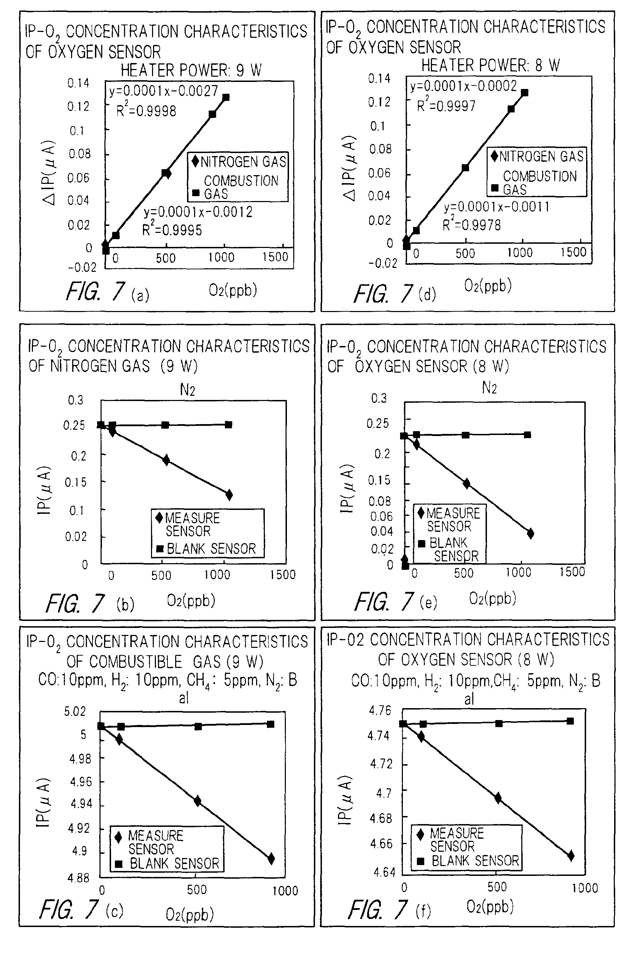

Oxygen was added to nitrogen and combustible gases in accordance with the above-mentioned procedure to values of oxygen concentration of 1.13 ppb, 12.2 ppb, 115 ppb, 570 ppb and 1,140 ppb, and the resultant gases were used as measurement gases. The result of measurement is illustrated in Figs. 7(a) to 7(f). Figs. 7(a) to 7(c) represent characteristics cases where the sensor was heated at 9 W, and Figs. 7(d) to 7(f) cover characteristics in cases where the sensor was heated at 8 W.

Figs. 7(b) and (e) illustrate pump current - oxygen concentration characteristics of the blank sensor and the measure sensor when the trace oxygen - added nitrogen gas flows with and without passage through the oxygen remover 41. Fig. 7(c) and (f) illustrate pump current - oxygen concentration characteristics of the blank sensor and the measure sensor when trace oxygen-added combustible gases (CO: 10 ppm, H₂: 10 ppm, CH₄: 5 ppm, and N₂: balance) flow with and without passage through the oxygen remover 41. Figs. 7(a) and 7(d) illustrate measured values showing the relationship between the oxygen concentration and a difference ΔIp obtained by subtracting the pump current of the measure sensor when the same measurement gas as in Figs. 7(b), (c), (e) and (f) not having passed through the oxygen remover 41 from the pump current of the blank sensor when the same measurement gas having passed through the oxygen remover 41. The result shows that while there are large differences in pump current value between nitrogen as and the combustible gas, there is almost no difference in the pump current between ΔIp caused by the presence or absence of the passage through the oxygen remover 41. Similarly, there is a difference in sensor temperature of 740°C and 800°C between the values of sensor heating power of 8 W and 9 W, there is almost no difference between 8 W and 9 W, although a difference in temperature is observed in pump current. This is attributable to the fact that ΔIp represents a quantity of oxygen removed in the oxygen remover 41, and this quantity of removed oxygen is not dependent upon the kind of gas or temperature. As is clear from this result, it is possible to rapidly and accurately measure the quantity of oxygen on a ppb level by using the measuring apparatus of the first aspect of the invention.

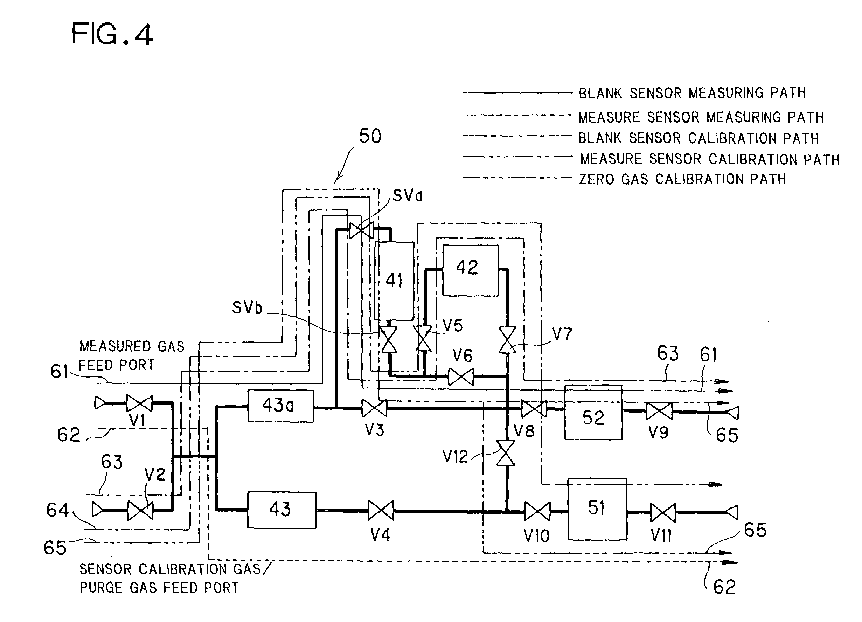

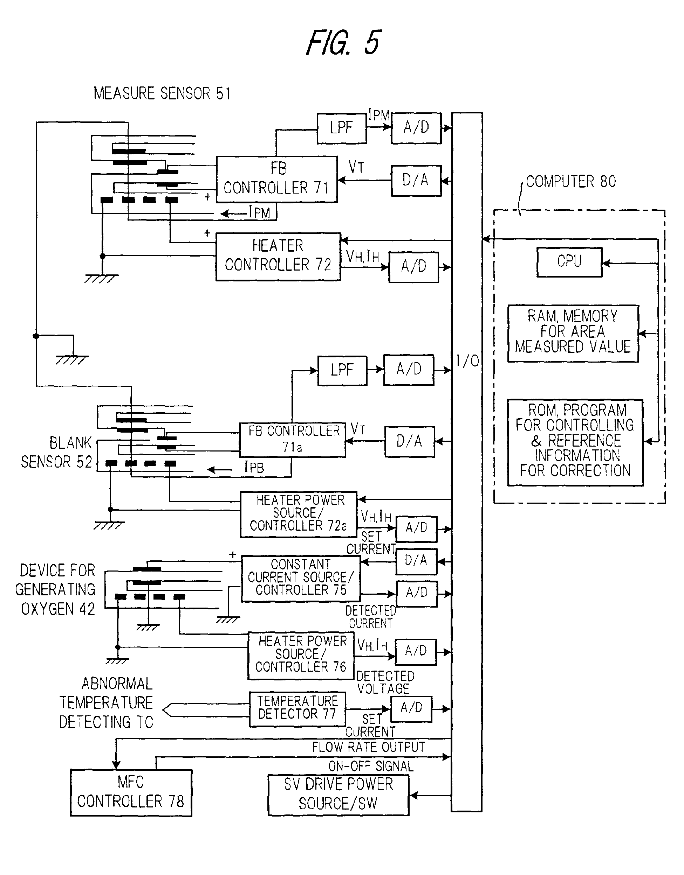

Fig. 4 illustrates a schematic view for explaining an outline of the configuration for another embodiment of the measuring apparatus of the first aspect of the invention. In this embodiment, two oxygen sensors are used, and serve independently as a blank sensor and a measure sensor, respectively. Fig. 5 illustrates major components of the measuring apparatus 50 shown in Fig. 4, in the form of a block diagram. In this embodiment, because two oxygen sensors are used, mass flow controllers are provided, corresponding to the oxygen sensors. It is needless to mention that a series of valves are provided so as to permit switching of the flow paths in response to the kind of gas flowing therethrough. In this embodiment, the branching mechanism would be operated to change the flow path in response to the kind of gas passing therethrough. Description is omitted since the other mechanisms are the same in principle as in the above.

In this measuring apparatus 50 as well, the measurement gas feed port and the feed port of nitrogen gas which is the sensor calibrating/purge gas are provided at the leading end of piping having the leading end branching into two which lead to a single flow path via valves attached to the individual flow paths. The single flow path branches again into two. In a flow path 61, the measurement gas passes through only the oxygen remover 41 and is directed to the blank sensor 52 by switching over the series of valves including the valves SVa, SVb, V6, V8 and V9 to open as prescribed via a second mass flow controller 43a; in a flow path 62, the measurement gas is directed directly to the measurement sensor 51 by opening the valves V4, V10 and V11, via the first mass flow controller 43; in a flow path 63 which is a first standard gas flow path, the sensor calibrating nitrogen gas flows through the second mass flow controller 43a and is directed through the three flow paths, i.e., through the oxygen remover 41 and the device for generating oxygen 42 to the blank sensor 52 by switching, as prescribed, the series of valves including the valves SVa, SVb, V5, V6 and V7; in the flow path 64 which is a second standard gas flow path, the gas passes through the oxygen remover 41 and the device for generating oxygen 42, and directed to the measure sensor 51; and in the flow path 65 which is a zero gas flow path, the gas passes through the oxygen remover 41 and directed to the blank sensor or the measure sensor. These flow paths are switched over as required.

Nitrogen gas which is purging and sensor calibrating gas passes through only the oxygen remover 41, resulting in a standard gas (oxygen concentration: below 1 ppb) by substantially completely removing oxygen contained in nitrogen gas. A fraction thereof is fed to the blank sensor to measure pump current at this point in time, and the remaining portion passes through the oxygen remover 41 and the device for generating oxygen 42, and a nitrogen gas from which oxygen has been substantially completely removed in the oxygen remover 41 (oxygen concentration: below 1 ppb; the same also for the subsequent cases) is obtained. Then, prescribed oxygen pump convert giving a desired oxygen concentration is fed to the device for generating oxygen 42 where nitrogen gas is adjusted so as to contain oxygen in a prescribed concentration. The gas is finally fed to the blank sensor where respective pump current values are measured. A calibration curve (relational formula) of oxygen concentration - pump current is prepared on the basis of this result, and the sensor is calibrated in accordance with the result.

When an oxygen concentration of at least 2 ppm in the measurement gas is previously predicted, the valves SVa and SVb should be closed because otherwise the oxygen remover 41 suffers from serious wear. In the case of the measuring apparatus 50 shown in Fig. 4, if the measurement gas is fed, the measurement gas flows directly into the measures sensor 51, and the measurement gas from which oxygen has been removed in the oxygen remover 41 flows into the blank sensor 52. The measurement sensor and the blank sensor simultaneously measure pump current, and the difference between the two pump current values corrected in terms of the individual sensor difference is continuously measured as the oxygen concentration of the measurement gas. This method is characterized in that, because it is a differential signal processing, it is resistant to a change in interference gas concentration and temperature. The oxygen corresponding to the combustible gas quantity measured by the measure sensor 51 and the blank sensor 52 (representing the quantity of oxygen just sufficient to cause complete combustion of the combustible gases) and oxygen for maintaining the oxygen concentration set electromotive force in the measuring duct are introduced into the measuring duct by means of the oxygen pump cell to cause combustion of the combustible gases in the measurement gas, thus performing operation for keeping the electromotive force in the measuring duct at the set value.

In the blank sensor 52, oxygen in the measurement gas has been almost completely removed in the oxygen remover 41. In the measure sensor 51, the measurement gas contains oxygen from beginning, and pump current is reduced by a current corresponding to the oxygen concentration contained from the beginning as compared with the blank sensor 52. In the embodiment shown in Fig. 4, therefore, in which two oxygen sensors are used, the pump current flowing to the sensors 51 and 52 is corrected, taking account of the difference between individual sensors in the gas diffusion quantity into the measuring duct 19. The difference after correction between the sensors represents the quantity of oxygen removed in the oxygen remover 41, thus permitting accurate and continuous measurement of the concentration of oxygen contained from the beginning in the measurement gas.

The trace oxygen measuring apparatus shown in Fig. 4 comprises a measured gas feed port, a sensor calibrating/ purge gas feed port, first and second mass flow controllers, an oxygen remover 41, a device for generating oxygen 42, and first and second oxygen sensors 51 and 52. The second mass flow controller 43a is provided in parallel with the first mass flow controller 43, and the second oxygen sensor 52 is provided in parallel with the first oxygen sensor 51. The first oxygen sensor 51 is a blank sensor, and the second oxygen sensor 52 is a measure sensor. The flow path on the blank sensor 52 side is arranged so that the blank sensor calibrating gas and the measure sensor calibrating gas are fed to the blank sensor 52 via the oxygen remover 41 and/or the device for generating oxygen 42 by means of the first mass flow controller 43 and a changeover valve. A feedback controller 71 may be provided for controlling the detecting cell electromotive force to a prescribed set voltage through oxygen supply by a pump cell provided in the oxygen sensor.

Furthermore, the trace oxygen measuring apparatus of this embodiment may be configured as follows. The first and second oxygen sensors are made of a plurality of solid electrolyte layers, and have a first air duct 12A, a second air duct 12B and a measuring duct 19 defined by the plurality of solid electrolyte layers. An oxygen discharge electrode 16 and a concentration detecting electrode 17 are provided in the measuring duct 19. There is provided an oxygen pump cell 10 formed via the solid electrolyte layers formed between the first air duct 12A and the measuring duct 19, from the oxygen feed electrode 15 formed in the first air duct 12A and the oxygen discharge electrode 16 formed in the measuring duct 19. The oxygen feed electrode 15 formed in the first air duct 12A and the oxygen discharge electrode 16 formed in the measuring duct 19 is for feeding pump current between these electrodes for controlling the concentration detecting cell electromotive force to a prescribed set value. It forms a pump cell for pumping oxygen in a quantity meeting the pump current value from the first air duct 12A. An air reference electrode 18 formed in the second air duct and a concentration detecting electrode 17 formed in the measuring duct 19 form a concentration detecting cell 13 via the solid electrolyte layers formed between the second air duct 12B and the measuring duct 19 to perform feedback operation and measure the concentration of oxygen in the measurement gas by means of pump current of the pump cell.

The measuring procedure of the oxygen concentration in the measurement gas using the above-mentioned measuring apparatus 50 will be described further in detail.

An example of measuring procedure in the measuring apparatus 50 shown in Fig. 4 is as follows. First, the procedure comprises the steps of opening the valve V2 of the sensor calibrating/purge gas feed port, turn on power to start the apparatus, causing the flow paths with nitrogen gas, then, causing the oxygen generator 42 to heat-run, and causing the oxygen sensor 51, the drive FB controller 71, the oxygen sensor 52 and the drive FB controller 71a to heat-run. An FB threshold voltage of, for example, 210 mV is given to the FB controllers 71 and 71a by an instruction of the computer. When the electromotive force of the concentration detecting cell 13 of the individual oxygen sensors 51 and 52 exceeds 210 mV, the oxygen pump is operated by feeding necessary current from the FB controllers 71 and 71a to the oxygen sensors 51 and 52, and oxygen is pumped up so that the concentration detecting cell electromotive force from the oxygen feeding air duct side to the measuring duct side becomes 210 mV. Then, the valves V1, V3, V4, V5, V7, V10 and V11 are closed, and the valves V2, SVa, SVb, V6, V8, and V9 are opened.

Deoxidized nitrogen gas is fed from the nitrogen gas feed port of the sensor calibrating/purge gas through the second mass flow controller 43a and via the third standard gas flow path 65 to the blank sensor 52. For the nitrogen gas not containing oxygen by the oxygen remover 41, pump current corresponding to the necessary quantity of oxygen pumped up by the oxygen pump is measured in the blank sensor 52. The pump current measured in this case is the pump current when the quantity of oxygen is substantially zero (oxygen quantity: under 1 ppb). The gas flow rate is controlled by the mass flow controllers 43 and 43a and the MFC controller 78 to a prescribed flow rate, and a measurement signal of gas flow rate is sent to the computer 80. Then, the valve V6 is closed, and the valves V5 and V7 are opened. Thus, pump current corresponding to the quantity of oxygen necessary for achieving a desired oxygen concentration is fed to the device for generating oxygen 42 by means of the constant current source/controller 42, and oxygen in a prescribed quantity is added. After confirming that standard gas of a desired oxygen concentration as flowed to the first standard gas flow path 63, by means of the time lapse or the constant condition of pump current flowing to the blank sensor 52, the result of pump current - oxygen concentration of the sensor 52 is stored in the computer 80, and the blank sensor is calibrated.

Then, the valves V5, V7, V8 and V9 are closed, and the valves V6, V10 and V11 are opened. Deoxidized nitrogen gas is fed from the feed port of nitrogen which is the sensor calibrating/purge gas, via the third standard gas flow path 65 to the measure sensor 51. For the nitrogen gas not containing oxygen through the oxygen remover 41, pump current corresponding to the oxygen in a necessary quantity pumped up by the oxygen pump is measured in the measure sensor 51. Pump current measured in this case is the pump current when the quantity of oxygen is substantially zero (oxygen quantity: under 1 ppb). Then, the valve V6 is closed, and the valves V5 and V7 are opened.

Thus, pump current corresponding to the quantity of oxygen necessary for achieving a desired oxygen concentration is fed by the constant current source/ controller 75 to the device for generating oxygen 42, and oxygen in a prescribed quantity is added to nitrogen gas. Outflow of the desired oxygen concentration gas into the second standard gas flow path 64 is confirmed by a time lapse or the constant condition of current flowing through the measure sensor 51. Thereafter, the result of measurement of pump current - oxygen concentration of the sensor 51 is stored in the computer 80, and the measure sensor is calibrated.

Then, the valves V2, V3, V5, and V7 are closed, and the valves V1, SVa, SVb, V4, V6, V8, V9, V10 and V11 are opened to feed measurement gas both to the blank sensor 52 and to the measure sensor 51. Supply to the blank sensor 52 is accomplished by feeding measurement gas from the measurement gas feed port, through the second mass flow controller 43a and the flow path passing through the oxygen remover 61 to the blank sensor 52. After confirming that the flow path passing through the oxygen remover 61 has been replaced by the measurement gas having passed through the oxygen remover 41, the pump current of the blank sensor 51 is measured and stored in the computer 80. Supply to the measure sensor 51 is accomplished by feeding the gas from the measurement gas feed port through the first mass flow controller 43 and the direct flow path 62, directly to the measure sensor 51. After confirming that the direct flow path 62 has been replaced by the measurement gas, the pump current of the measure sensor 51 is measured and stored in the computer 80. The computer 80 conducts correction of individual difference between the blank sensor 52 and the measure sensor 51, and continuously determine the oxygen concentration in the measurement gas from the difference in pump current. As required, it is possible to calculate the oxygen consumption by the combustible gas ;from the pump current of the blank sensor 52. Finally, the process is completed by turn off power.

When continuously measuring oxygen in the measurement gas, calibration by the standard gas may be omitted. In this case, the valves V2, V3, V5, V7 and V12 are closed, and the valves V1, SVa, SVb, V4, V6, V8, V9, V10 and V11 are opened, and the steps ending with calculation in compliance of an instruction from the computer 80 is repeated.

When the measurement result of oxygen concentration should be corrected, the correction is performed on the computer. The operation is usually carried out in accordance with a program incorporated into the computer. It is of course possible to make various modifications in response to the object of measurement and measuring conditions. A desired measurement can be made by previously incorporating such a modification program into the computer.

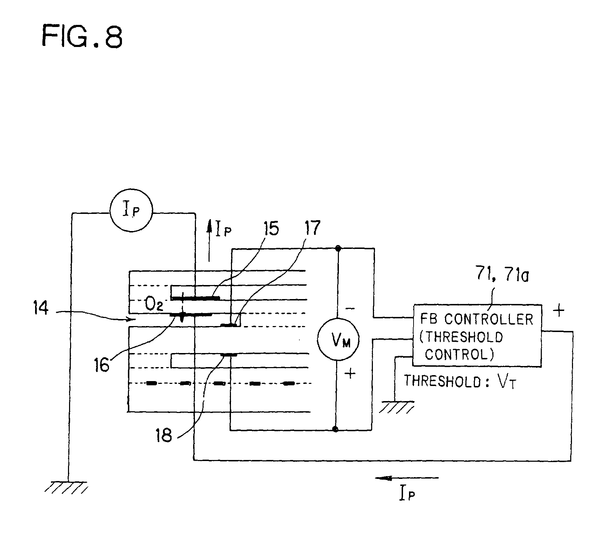

Fig. 8 is a descriptive view illustrating operation of the oxygen sensor (measure sensor 51 and blank sensor 52) shown in Fig. 1. First, a target value V_{T} is set for the electromotive force V_{M} between the concentration detecting electrode 17 and the air reference electrode 18. This target value V_{T} may be, for example, about 240 mV or less at an oxygen concentration of 1 ppm, for example, 210 mV.

When the electromotive force V_{M} is larger than the target value V_{T}, pump current Ip is fed from the FB controllers 71 and 71a to the oxygen pump cell (between the oxygen feed electrode 15 and the oxygen discharge electrode 16) to feed oxygen from the oxygen feed electrode 15 to the oxygen discharge electrode 16, and feedback control is performed so that a prescribed electromotive force V_{T} is achieved by burning combustible gases, if any, in the measuring duct 14. On the contrary, if the electromotive force V_{M} is smaller than the target value V_{T}, pump current I_{B} is fed so as to cause movement of oxygen from the oxygen discharge electrode 16 to the oxygen feed electrode 15 to pump out oxygen in the measuring duct to the first air duct. Thus, the polarity of pump current Ip becomes reverse with the target value V_{T} as a boundary. The shortage or excess of oxygen necessary to achieving the target value V_{T} of the oxygen concentration in the measuring duct including combustion of combustible gases can be determined from the polarity and magnitude of pump current. The quantity of oxygen removed by the oxygen remover 41 is determined from the difference in pump current between removal and non-removal of oxygen in the measurement gas in a single sensor, and this represents the oxygen concentration in the measurement gas.

Pump current I_{PB} measured by the blank sensor 52 and pump current I_{PM} measured by the measure sensor 51 are corrected, taking account of the difference between individual sensors, thus determining the difference in pump current.

If the resultant pump current is I_{P}, oxygen required for combustion of combustible gases, I_{P1}, oxygen present in the measurement gas, I_{P2}, and oxygen necessary for achieving a set oxygen concentration in the measuring duct, I_{P3}, the pump current for the measuring sensor would be expressed by: I_{PM} = I_{P1} - I_{P2} + I_{P3}, and because oxygen in the measurement gas is removed in the blank sensor, this is rewritten as: I_{PB} = I_{P1} + I_{P3}. This results therefore in: I_{PB} - I_{PM} = I_{P2}. Since the difference in pump current between the blank sensor and the measure sensor is based on oxygen corresponding to the oxygen concentration contained in the measurement gas from the beginning, it is possible to more accurately know the oxygen concentration in the measurement gas while excluding the effect of interference gas such as combustible gases, by using the above-mentioned measuring method.

In the measuring apparatus 50, measurement of oxygen concentration is carried out for the measurement gas fed at almost the same timing to the measure sensor 51 and the blank sensor 52. It is therefore applicable even when the measurement gas composition varies during a short period of time. On the other hand, in the measuring apparatus 40, in which the oxygen sensor 10 is used as the measure sensor and the blank sensor in the time-sharing manner, the measuring timing shifts between the blank sensor and the measure sensor. A measurement gas of which the gas composition varies during a short period of time, is not suitable as an object of measurement. This method is suitably applicable only for measurement gases of which the gas composition is rather stable.

According to the trace oxygen measuring apparatus and the measuring method of the first aspect of the present invention, as described above, it is easy to downsize the apparatus, and because a ZrO₂ oxygen sensor excellent in maintainability and service life is used in an apparatus of a simple configuration, a good operability is available. Furthermore, there are available excellent advantages such as rapid measurement of an accurate trace oxygen concentration excluding the effect of interference gases such as combustible gases. It is also possible to change the ZrO₂ oxygen sensor design into various manners, thus permitting achievement of multi-range and higher-accuracy apparatus. It provides a wide range of applications, because calibration of the oxygen sensor is easy, in addition to the above advantages.

The second aspect of the invention will now be described in detail with reference to the drawings. In the following description of the invention, the components and units having the same or similar functions as in the trace oxygen measuring apparatus of the first aspect of the invention will be represented by the same reference numerals in principle.

In this specification of this application, the term "a layer" as to the number of solid electrolyte layers does not always means a layer composed of a single layer, but include a multiple layers of the same or similar functions if such layers forms a single assembly.

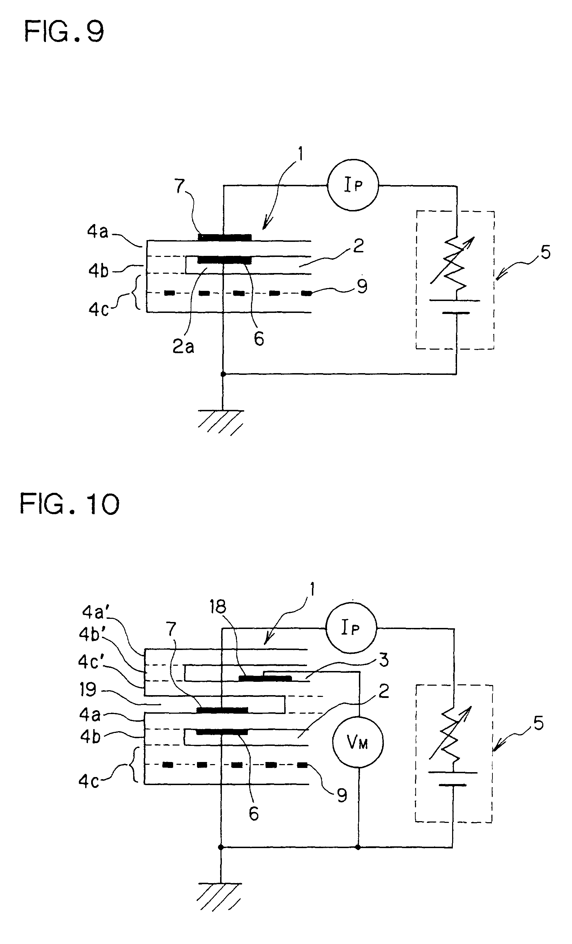

The device for generating trace oxygen 42 of the second aspect of the invention comprises a plurality of solid electrolyte layers, and as shown in Fig. 9, has a first air duct 1 which is a vacancy defined by the solid electrolyte layers 4a, 4b and 4c forming three continuous layers at least at ends thereof, an oxygen feeding duct 2a comprising an oxygen feed electrode 6 formed in the air duct, and an oxygen pump cell comprising an oxygen discharge electrode 7 provided on the surface of the upper layer 4a of the solid electrolyte layers forming the above three layers, and an oxygen feed electrode 6 formed in the air duct 2. A constant current source/controller 5 is arranged between the oxygen feed electrode and the oxygen discharge electrode 7 so as to permit flow of prescribed current.

Fig. 9 is a schematic view illustrating the basic structure of the device for generating a race oxygen 42 of the second aspect of the invention. In this example, while the solid electrolyte layer 4a having the oxygen discharge electrode 7 provided thereon and one end are located at the same position, a solid electrolyte layer 4b smaller than the layer 4a by a distance corresponding to the length of the first vacancy, and a first vacancy defined by the solid electrolyte layer 4c having the same length as the layer 4a are formed. The solid electrolyte layer 4b regulating the opening height of the air duct having the oxygen feed electrode 6 provided is formed by a single solid electrolyte layer. By composing this solid electrolyte layer 4b with a plurality of layers, it is possible to increase the oxygen diffusing quantity of the oxygen feed duct, and increase the constant current allowance for generating oxygen. Particularly, when achieving a high oxygen concentration from a high gas flow rate, the solid electrolyte layer 4b can be composed of a plurality of layers. It was sufficiently possible, in an example of a thick-film ZrO₂ porcelain, to build a 10 mA/eight layers (oxygen generation: about 38 µl/min) by using a tape of about 200 µm/layer.

Fig. 10 is a schematic view illustrating the basic structure of the device for generating trace oxygen 42 of another embodiment of the second aspect of the invention. In this embodiment, a second air duct 3 is formed above the first air duct via a discharge duct 19a. While this second air duct 3 has an end located at the same position as the solid electrolyte layer 4a', it is defined by the solid electrolyte layer 4b' smaller than the layer 4a' by a length corresponding to the length of the second vacancy and a solid electrolyte layer 4c' having the same length as the layer 4a'. The discharge duct 19a is formed so that the opening surface is opposite to the first and second ducts. An oxygen discharge electrode is provided in the discharge duct 19a, and an oxygen feed electrode 6 is provided in the first air duct 2. An air reference electrode 18 is provided in the second air duct 3. The oxygen feed electrode 6 and the oxygen discharge electrode 7 form an oxygen pump cell, and the oxygen feed electrode 6 and variation of produced electromotive force form a detecting cell for monitoring the decrease in the oxygen concentration in the air feed duct. That is, upon occurrence of a decrease in oxygen concentration in the air feed duct, an electromotive force is generated in the detecting cell, and maintenance of accuracy of the device for generating a trace oxygen and prevention of deterioration thereof can be achieved by monitoring this electromotive force and maintaining it within an allowable range.

The device for generating a trace oxygen 42 usually made of ZrO₂ porcelain as the solid electrolyte is suitably applicable. This solid electrolyte serves also as a partition which separates and regulates trace oxygen gas generated in the air duct 2. The solid electrolyte should preferably have a high oxygen ion conductivity. In the case of ZrO₂, it is possible to change the ion conductivity by acting on the kind of solute material and the quantity thereof, and thus to use a composition suitable for a particular purpose.

As such ZrO₂ porcelain, stabilized ZrO₂ or partially stabilized ZrO₂ prepared through solid-solution of various stabilizing materials such as yttria (Y₂O₃), magnesia (MgO), calcia (CaO) and ceria (CeO₂) are suitably applicable.

In the embodiment shown in Fig. 9, the oxygen discharge electrode 7 provided on the solid electrolyte layer 4a and the oxygen feed electrode 6 provided in the first air duct via the solid electrolyte layer 4a in pair serve as an oxygen pump cell. What is important here is that the first air duct 2 is designed to contain oxygen in a sufficient quantity. In the embodiment shown in Fig. 10, an air reference electrode 18 forming a detecting cell which monitors a decrease in the oxygen concentration of the air feed duct is provided in the second vacancy 3, oppositely to the oxygen feed electrode 6. It is therefore possible to monitor a decrease in the oxygen concentration from a change in produced electromotive force of the air reference electrode 18.

These electrodes 6, 7 and 18 must have a satisfactory electron conductivity, and a high oxygen ion catalytic property is another important property. For example, the oxygen ion catalytic property in the oxygen discharge electrode 7 means the property of taking electrons from oxygen ions moving from the oxygen feed electrode 6 to the solid electrolyte, and releasing them as oxygen molecules to outside the oxygen discharge electrode. On the other hand, the oxygen ion catalytic property for the oxygen feed electrode 6 and the air reference electrode 18 means in contrast a property of ionizing oxygen ions in the air and incorporating them in the solid electrolyte.

As electrode materials excellent in such a property, gold (Au) and platinum (Pt) are suitably applicable. Usually, considering the number of printing processes and the baking temperature of electrodes, it is desirable to use the same electrode material. These electrodes should preferably be porous and formed so as to form many triple point (three-phase surfaces) where the gas phase, electrode and solid electrolyte are in contact with each other. Therefore, a thermet electrode comprising Pt and ZrO₂ is suitably applicable.

As shown in Figs. 9 and 10, there is arranged a heater 9 heated by a heating unit (not shown) formed so as to maintain a prescribed temperature. By raising temperature of the device for generating a trace oxygen 42 and maintaining at a prescribed temperature, ion conductivity of the solid electrolyte layers made of ZrO₂ present between the both electrodes is improved, the inner resistance between the both electrodes is reduced, thus permitting stabilization of the generated oxygen concentration.

The constant current source/controller 5 which is a control function for generating oxygen in a prescribed quantity will now be described. This constant current source/controller 5 is configured so as to feed a constant current corresponding to the generated quantity of oxygen (oxygen concentration if a gas flow rate is determined) between the oxygen discharge electrode 7 and the oxygen feed electrode 6 for generating oxygen in a prescribed quantity. The allowable current which can be fed, depending upon the number of solid electrolyte layers forming the wall surface 4b of the air duct, is about 1.2 mA (generated oxygen quantity: about 4.5 µl/min) when the number of layer is one, using a tape of about 200 µm/layer. Eight layers give oxygen quantity of about 10 mA (generated oxygen quantity: 38 µl/min). The constant current fed is controlled by a signal sent to the constant current source/controller 5 in accordance with the generated quantity control program of the computer 80 previously incorporated into the oxygen generator.

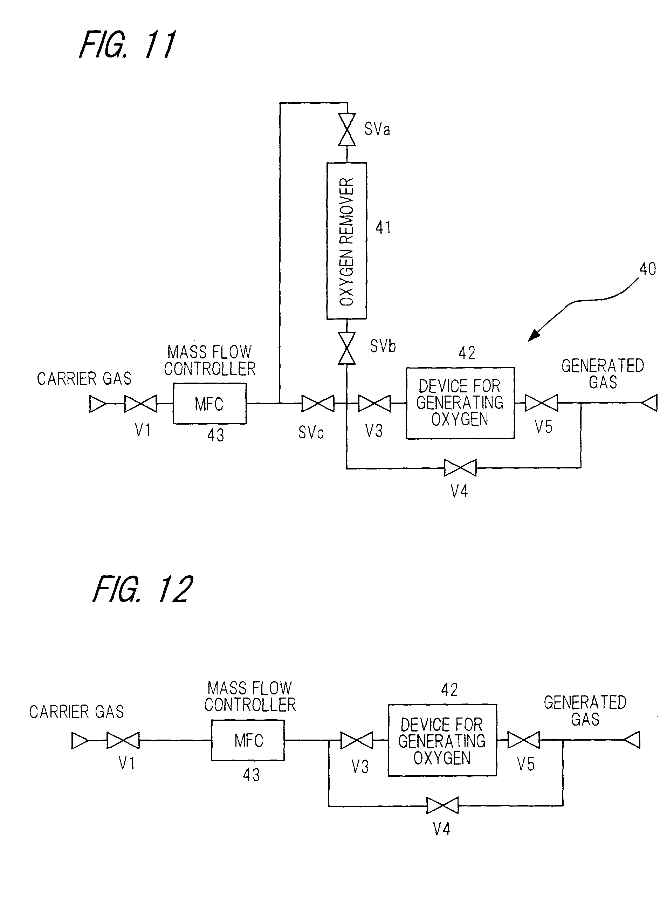

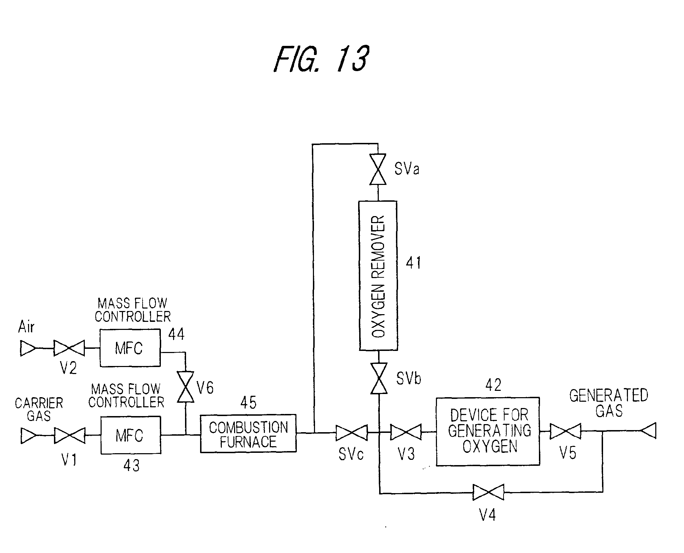

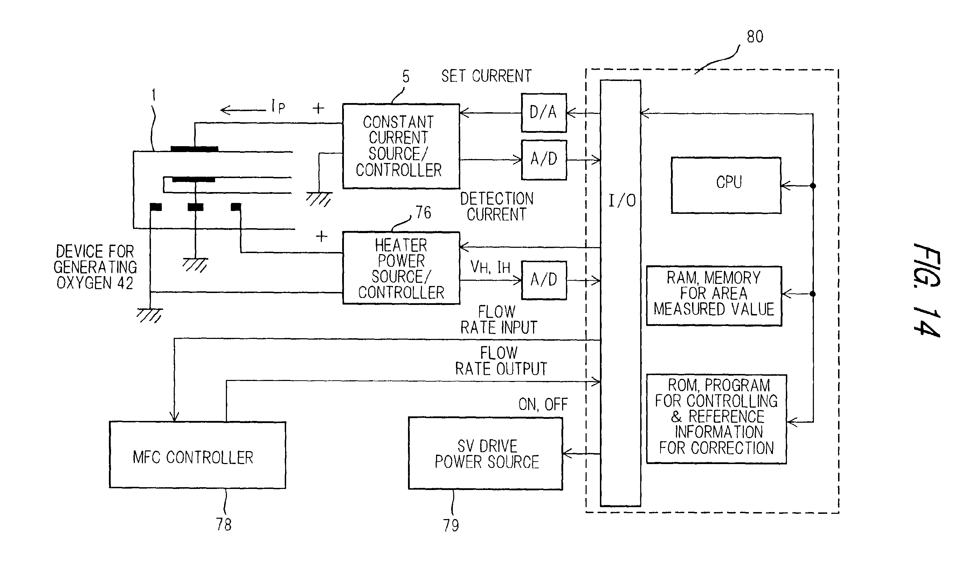

For a case where the device for generating a trace oxygen 42 of the second aspect of the invention is used in the trace oxygen measuring apparatus 40 of the first aspect of the invention, configuration diagrams are shown in Figs. 11, 12 and 13, and a block diagram is illustrated in Fig. 14.

As shown in Fig. 11, the basic configuration comprises a carrier gas feed port, a mass flow controller 43, an oxygen remover 41, and a device for generating a trace oxygen 42. As shown in Fig. 14, the a device for generating a trace oxygen 42 is controlled at a constant temperature by the heater power source/controller 76, and the computer 80 calculates a quantity of oxygen and current to be fed meeting a set oxygen concentration with reference to a gas flow rate signal from the MFC controller 78, controls the constant current source/controller 5, and feeds oxygen to a device for generating a trace oxygen 42.

In order to actuate the a device for generating a trace oxygen of the invention in the measuring apparatus 40, the standard gas serving as the carrier gas, usually nitrogen gas, passes through the mass flow controller 43 so as to contain oxygen in a desired concentration. The gas is first directed to the oxygen remover 41 by switching over and valves SVa, SVb and SVc. In the oxygen remover 41, oxygen is removed so as to achieve a concentration lower than 1 ppb, and then, by opening the two valves provided before and after the device for generating a trace oxygen 42 previously switched over, the gas passes through the device for generating a trace oxygen 42, adjusted so as to contain oxygen in a prescribed concentration, and regulated mainly by the opening/closing of the valves V1, V3, V4, V5, SVa, SVb and SVc. Bypass flow paths of SVc and V4 are provided in the oxygen remover 41 and the device for generating a trace oxygen 42.

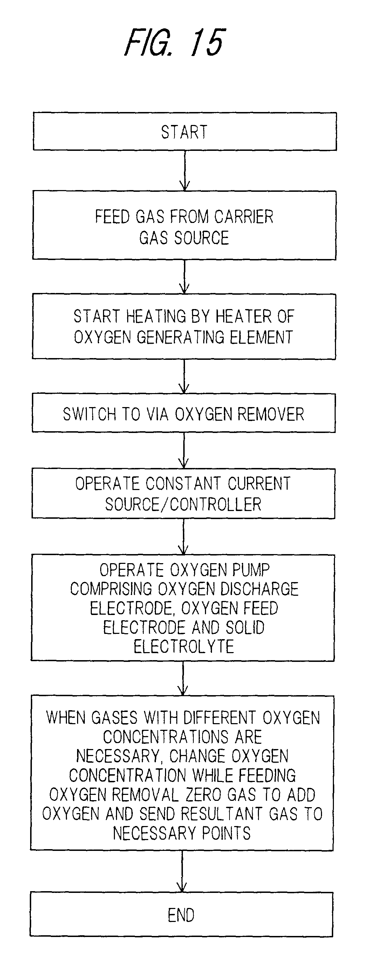

A method of generating trace oxygen of the second aspect of the invention will now be described with reference to the flowchart shown in Fig. 15.

The generating process comprises the step of first opening the valves V1, SVc, V3 and V5 to feed a carrier gas, such as nitrogen gas. The device for generating a trace oxygen 42 is heated by the heater power source/controller. When the pipe is sufficiently purged with the carrier gas, the valves SVa and SVb are opened and the valve SVc is closed.

The constant current source/controller operable by the instruction of the computer is actuated to feed constant current to the oxygen pump cell comprising the oxygen feed electrode and the oxygen discharge electrode so as to generate oxygen in a prescribed quantity, to operate the oxygen pump. By so doing, zero gas from which oxygen has been removed through the oxygen remover is sent to the oxygen pump, where the gas receives oxygen fed from the oxygen pump, and the resultant gas containing trace oxygen in a prescribed quantity to the necessary points. At this point in time, in order to obtain an oxygen concentration of 0 to 2 ppm at a gas flow rate of 2 l/min, the constant current source/controller should be configured so as to be capable of feeding current of 10 nA to 1.2 mA as constant current. The fed current is controlled, for example, by means of a program previously incorporated into the computer. Because the quantity of generated oxygen is controlled with a quantity of current fed to the device for generating a trace oxygen 42, it is possible to generate oxygen on the ppb level.

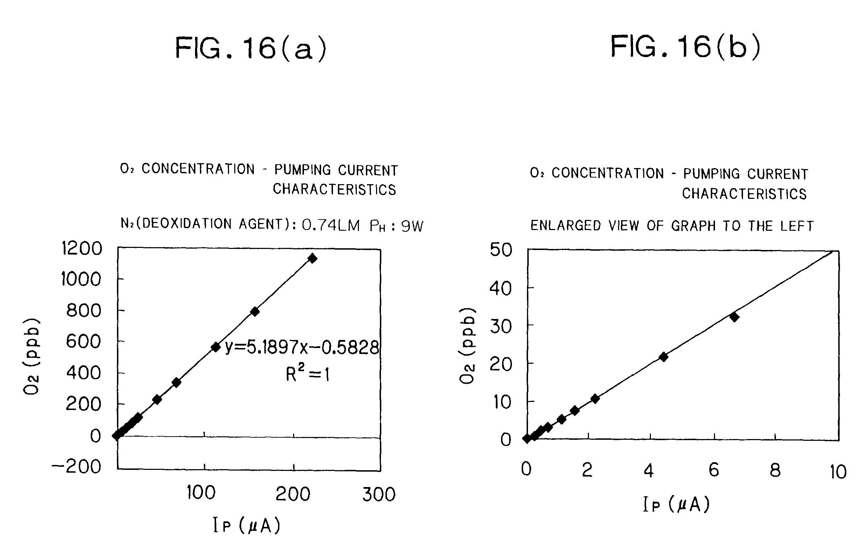

In a case where the device for generating a trace oxygen 42 schematically shown in Fig. 9 is used to feed prescribed current from the constant current source/controller, the relationship between the constant current at a carrier gas flow rate of 0.74 l/min and the concentration of the generated oxygen (ppb) is illustrated in Figs. 16(a) and 16(b). The room temperature for this experiment was 23°C, with heating conditions of heater including 8.60 V and a power consumption of 9.02 W. Fig. 16(a) shows the O₂ concentration - pumping current characteristics when generating oxygen in a concentration from 0 to 1,140 ppb; and Fig. 16(b) shows the O₂ concentration - pumping current characteristics when generating oxygen in a concentration from 0 to 50 ppb. Particularly as is clear from Fig. 16(b), it is well possible to generate oxygen in an accurate quantity even within a range of from 0 to 50 ppb.

In the case that the device for generating a trace oxygen, the trace oxygen generator using the devise, and the method generating for a trace oxygen according to the second aspect of the present invention, as described above, it is possible to very accurately and easily generate trace oxygen. The apparatus itself can be downsized easily. Because the apparatus is simple in structure, using a ZrO₂ sensor excellent in maintainability and service life, a high operability is available. Therefore, the present inventive device is useful not only as a calibrating gas generator for a trace oxygen measuring apparatus, but also as an oxygen generator used for testing the effect of trace oxygen.

The present invention discloses a trace oxygen measuring apparatus comprising a trace oxygen generating unit capable of generating oxygen in an arbitrary amount within a range of from 1 to 2 ppm, a concentration detecting cell configured so as to ensure followup of Nernst's formula, an opposite pump electrode provided in a special air duct, and a concentration detecting cell and a pump cell electrode independently provided; and a trace oxygen generating apparatus used such trace oxygen measuring apparatus.

## Claims

1. A trace oxygen measuring apparatus being provided with at least one limiting current type oxygen sensor having an oxygen pump cell comprising an oxygen ion conductive solid electrolyte and a metal electrode, and a concentration detecting sensor, said limiting current type as a blank sensor or a measure sensor; wherein:
when said limiting current type oxygen sensor serves as a blank sensor, an oxygen concentration in a deoxidizing measuring gas obtained by feeding a measurement gas through an oxygen remover is measured by means of pump current of a limiting current type sensor:
when said limiting current type oxygen sensor serves as a measure sensor, an oxygen concentration in the measurement gas is measured by means of pump current of the limiting current type sensor; and
said apparatus has a mechanism for calculating difference in pump current between the measure sensor and the blank sensor as an oxygen concentration contained in said measurement gas.

2. A trace oxygen measuring apparatus according to claim 1, which is provided with a branching mechanism for branching said measurement gas; wherein:
a measurement gas passes through said oxygen remover by action of said branching mechanism and is then fed to said blank sensor; and
another measurement gas is directly fed to said measure sensor by action of said branching mechanism.

3. A trace oxygen measuring apparatus according to claim 1; wherein there is provided a switching mechanism for measuring values of pump current as a blank sensor and a measure sensor; said mechanism capable of switching over time wise in the following manner;
a deoxidized measurement gas after passing through said oxygen remover is fed during a period of time, and
said measurement gas is fed directly to said oxygen sensor during another period of time.

4. A trace oxygen measuring apparatus according to claim 1, wherein there are provided two limiting current type oxygen sensors each having an oxygen pump cell comprising said oxygen ion conductive solid electrolyte and a metal electrode, and a concentration detecting cell, and wherein one of the limiting current type oxygen sensor is used as a blank sensor, and an other limiting current type oxygen sensor is used as a measure sensor.

5. A trace oxygen measuring apparatus according to any one of claims 1 to 4, wherein there is provided a feedback controller for controlling an electromotive force of the concentration detecting cell by feeding or discharging oxygen with current energizing the oxygen pump of said limiting current type oxygen sensor at a prescribed set voltage.

6. A trace oxygen measuring apparatus according to claim 5, wherein the set voltage of the electromotive force of the concentration detecting cell in said feedback controller is controlled to a voltage of up to 240 mV which corresponds to an oxygen concentration range of at least 2 ppm ensuring followup of the electromotive force - oxygen concentration characteristics of the concentration detecting cell to Nernst's formula.

7. A trace oxygen measuring apparatus according to any one of claims 5 and 6, further comprising a special air duct communicating with open air as an oxygen source necessary for feedback control effected by said feedback controller.

8. A trace oxygen measuring apparatus according to any one of claims 1 to 7, wherein:
said oxygen sensor is formed with a plurality of solid electrolyte layers; and
a first air duct, a second air duct and a measuring duct defined by said plurality of solid electrolyte layers;
said measuring duct has an oxygen discharge electrode and a concentration detecting electrode;
an oxygen pump cell formed of an oxygen feed electrode formed in said first air duct, and an oxygen discharge electrode formed in said measuring duct via the solid electrolyte layers formed between said first air duct and said measuring duct; and
a concentration detecting cell having an air reference electrode formed in said second air duct and a concentration detecting electrode formed in said measuring duct, via the solid electrolyte layers formed between said second air duct and said measuring duct; and
a mechanism for measuring the oxygen concentration in the measurement gas by measuring the oxygen pump current during feedback control through operation of the oxygen pump so that the electromotive force of said concentration detecting cell becomes a prescribed set voltage.

9. A trace oxygen measuring apparatus according to claim 8, wherein, in said oxygen sensor, the electrode present in the first air duct is an air reference electrode; the electrode opposite thereto is a concentration detecting electrode; the electrode present in the second air duct is an oxygen feed electrode; and the electrode opposite thereto is an oxygen discharge electrode.

10. A method of measuring the trace oxygen concentration in a measurement gas containing trace oxygen by means of an oxygen sensor, comprising the steps of:
using said oxygen sensor, wherein:
said oxygen sensor is formed with a plurality of solid electrolyte layers; and
a first air duct, a second air duct and a measuring duct defined by said plurality of solid electrolyte layers;
said measuring duct has an oxygen discharge electrode and a concentration detecting electrode;
an oxygen pump cell formed of an oxygen feed electrode formed in said first air duct, and an oxygen discharge electrode formed in said measuring duct via the solid electrolyte layers formed between said first air duct and said measuring duct;
a concentration detecting cell having an air reference electrode formed in said second air duct and a concentration detecting electrode formed in said measuring duct, via the solid electrode layers formed between said second air duct and said measuring duct; and
a mechanism for measuring the oxygen concentration in the measurement gas by measuring the oxygen pump current during feedback control through operation of the oxygen pump so that the electromotive force of said concentration detecting cell becomes a prescribed set voltage;
controlling the electromotive force set voltage of the concentration detecting cell in feedback control of said oxygen sensor to a prescribed voltage of up to 240 V which corresponds to an oxygen concentration range of at least 2 ppm ensuring followup of Nernst's formula of the concentration detecting cell electromotive force - oxygen concentration characteristics; and
feeding oxygen necessary for achieving a set oxygen concentration in the measuring duct from a special oxygen feed air duct communicating with open air.

11. A method of measuring the trace oxygen concentration in a measurement gas containing a combustible gas and trace oxygen by means of an oxygen sensor, comprising the steps of:
using said oxygen sensor, wherein:
said oxygen sensor is formed with a plurality of solid electrolyte layers; and
a first air duct, a second air duct and a measuring duct defined by said plurality of solid electrolyte layers;
said measuring duct has an oxygen discharge electrode and a concentration detecting electrode;
an oxygen pump cell formed of an oxygen feed electrode formed in said first air duct, and an oxygen discharge electrode formed in said measuring duct via the solid electrolyte layers formed between said first air duct and said measuring duct; and
a concentration detecting cell having an air reference electrode formed in said second air duct and a concentration detecting electrode formed in said measuring duct, via the solid electrode layers formed between said second air duct and said measuring duct;
using at least one such oxygen sensor having a mechanism for measuring the oxygen concentration in the measurement gas by measuring the oxygen pump current during feedback control through operation of the oxygen pump so that the electromotive force of said concentration detecting cell becomes a prescribed set voltage;
measuring the oxygen concentration of the measurement gas from which oxygen has been removed through the oxygen remover by means of the pump current value of the oxygen sensor; and
calculating the difference between the first measured oxygen pump current and the second measured oxygen pump current, as the oxygen concentration in the measurement gas, by measuring the oxygen concentration of the measurement gas, not having passed through the oxygen remover, by means of the pump current of the oxygen sensor.

12. A method of measuring trace oxygen according to claim 11, comprising the step of feeding the measurement gas through the oxygen remover or not through the same by operating a switching mechanism, to the oxygen sensor.

13. A method of measuring trace oxygen according to claim 11, comprising the steps of:
using two oxygen sensors;
measuring the oxygen concentration of the measurement gas from which oxygen has been remove through the oxygen remover by means of pump current of the first oxygen sensor; and
calculating, as the oxygen concentration in the measurement gas, the difference between pump current of the first oxygen sensor and pump current of the second oxygen sensor by measuring the oxygen concentration of the measurement gas, not having passed through the oxygen remover, by means of pump current of the second oxygen sensor.

14. A device for generating oxygen in a trace amount comprising
a plurality of solid electrolyte layers;
an oxygen feed duct comprising a first air duct which is a vacancy defined by the solid electrolyte layers forming three continuous layers at least at an end thereof and an electrode feed electrode formed in the first air duct; and
an oxygen pump cell comprising an oxygen discharge electrode provided on the surface of the upper solid electrolyte layer forming said three layers, and an oxygen feed electrode formed in said first air duct;
wherein a constant current source/controller is arranged between the oxygen discharge electrode and the oxygen feed electrode so that prescribed current flows therebetween.

15. A device for generating oxygen in a trace amount according to claim 14, comprising
a second air duct and an air reference electrode provided in said second air duct;
wherein said air reference electrode forms a detecting cell which monitors a decrease in oxygen concentration caused by oxygen feed in the oxygen feed duct through a change in electromotive force by measuring electromotive force between the air reference electrode and the oxygen feed electrode in said oxygen feed duct.

16. A trace oxygen generating apparatus comprising a plurality of solid electrolyte layers comprising:
an oxygen feed duct comprising a first air duct, which is a vacancy defined by solid electrolyte layers forming three continuous layers at least at an end thereof, and an oxygen feed electrode formed in said first air duct; and
an oxygen pump cell comprising an oxygen discharge electrode provided on the surface of an upper layer of the solid electrolyte layers forming said three layers, and an oxygen feed electrode formed in said first air duct;
wherein a constant current source/controller is arranged between the oxygen discharge electrode and the oxygen feed electrode so that prescribed current flows therebetween.

17. A trace oxygen generating apparatus according to claim 16, comprising
a second air duct and an air reference electrode provided in said second air duct in addition to said first air duct;
wherein said air reference electrode forms a detecting cell which monitors a decrease in oxygen concentration caused by oxygen feed in the oxygen feed duct through a change in electromotive force by measuring electromotive force between the air reference electrode and the oxygen feed electrode in said oxygen feed duct.

18. A method of generating trace oxygen comprising the steps of:
feeding constant current to the oxygen pump cell comprising an oxygen feed electrode and an oxygen discharge electrode by activating a constant current source/controller so as to generate prescribed oxygen and operating an oxygen pump;
sending a gas from a carrier gas source to the oxygen pump;
receiving oxygen fed from the oxygen pump; and
feeding a carrier gas added with the resultant trace oxygen in a prescribed amount to a necessary point.

19. A method of generating trace oxygen comprising the steps of:
feeding constant current to an oxygen pump cell comprising an oxygen feed electrode and an oxygen discharge electrode by operating a constant current source/controller so as to generate prescribed oxygen, and operating an oxygen pump;
sending zero gas which is a carrier gas from a carrier gas source from which oxygen has been removed through an oxygen remover using a deoxidizer;
receiving oxygen fed from the oxygen pump; and
feeding a gas containing trace oxygen in a resultant prescribed amount to places requiring such gas.

20. A method of generating trace oxygen comprising the steps of:
operating a constant current source/controller, and operating an oxygen pump by feeding constant current to an oxygen pump cell comprising an oxygen feed electrode and an oxygen discharge electrode so as to generate prescribed oxygen;
adding oxygen in a slight amount to a carrier gas from a carrier gas source, heating the resultant mixture to burn the combustible fraction, removing oxygen in an oxygen remover using a deoxidizer or the like, and sending a zero gas from which the combustible fraction and oxygen have been removed to the oxygen pump side;
receiving oxygen fed from the oxygen pump; and
feeding a gas containing trace oxygen in a resultant prescribed amount to laces requiring such gas.
